# EUROPEAN PATENT SPECIFICATION

(11) **EP 3 795 645 B1**
(45) Date of publication and mention of the grant of the patent: **14.02.2024**
(21) Application number: 20194758.7
(22) Date of filing: 07.09.2020
(51) Int. Cl.: C09C 1/00, C08K 9/02, C09D 1/00, C09D 7/61, C09D 7/40, C09D 7/62

(54) **PIGMENTS**
PIGMENTE
PIGMENTS

(30) Priority: 20.09.2019 EP 19198681
(43) Date of publication of application: 24.03.2021
(73) Proprietor: Merck Patent GmbH, 64293 Darmstadt (DE)
(72) Inventor: Schmitt, Adeliene, 69121 HEIDELBERG (DE); Gumsheimer, Udo, 67256 WEISENHEIM A. S. (DE)
(74) Representative: Merck Patent Association

(56) References cited:
- DE-A1-102014 018 276
- US-B2- 6 751 022
- US-B2- 8 657 431

## Description

The present invention relates to pigments based on particles which are coated with at least one layer which consists of a mixture of amorphous carbon and nanocrystalline graphite and to the use of these pigments in paints, plastics, industrial coatings, automotive coatings, printing inks and cosmetic formulations.

Currently dark blue/green/grey to black shades are achieved by the use of inorganic absorptive pigments, e. g., Prussian Blue (in case of dark blue shades), chromium oxide (in case of dark green shades), spinel or hematite-type black iron-oxide or cobalt-oxide, copper-manganese-iron-oxide, copper-chrome oxide and manganese-iron-oxide. Furthermore, graphite or graphite-like pigments and carbon black pigments are commercially available and may be applied in physical blends/mixtures to assist creating dark coloured shades as well.

Dark blue, dark green, grey to black effect pigments are commercially available and commonly produced by the precipitation of dark coloured copper oxides or iron oxides, e.g. Fe₃O₄, Co₃O₄ or FeTiOs, on platelet shaped substrates, e. g. natural or synthetic mica, glass flakes or Al₂O₃ flakes. Commercially available black or dark-grey pigments are produced by precipitation methods.

However, the dark-grey blue/green to black effect/pearl pigments exhibit significant disadvantages which restrict their use in some applications:
- Prussian blue and chromium oxide and cobalt containing pearl pigments are not allowed for the use in cosmetic applications due to the known allergenic property of heavy metals and cobalt-ions;
- Black iron-oxide coated pearl pigments may show magnetic effects, which are not favourable in some coatings applications;
- Ilmenite containing pearl pigments very often show intrinsically brownish absorptive colour, which demands further colour adjustment
in applications where neutral grey tones are targeted. Furthermore, ilmenite containing pearl pigments are not allowed in cosmetic formulations.

Instead of heavy metal oxide pigments carbon black or graphitan containing pigments can be used when it comes to creating a black pearl effect.

Carbon black containing pigments are known from the prior art, for example from DE-AS 11 65 182, DE 25 577 96 A1, DE 41 25 134 A1 and are prepared by applying carbon from an aqueous solution using surface-active auxiliaries or by pyrolysis of organic compounds. US 6,751,022 B2 teaches color shifting carbon-containing interference pigments but not dark metal effect pigments.

However, particles or effect pigments mixed with pure carbon black or graphitan show a non-attractive gloss. If a dark pearl effect is to be imitated by carbon black or graphitan, interference pigments must be blended with carbon black particles or graphitan particles physically. These physical blends however, are likely to segregate in certain (e. g. cosmetic) applications, which is prevented by adding dispersion and rheological additives.

Non-metallic interference pigments based on flake-form non-metallic supports which are coated with a layer which comprise crystalline carbon layer in the form of graphite-like and/or graphene are known from US 2017/0321057 A1. These interference pigments have the disadvantages that they are electrically conductive pigments and that they do not have a very sufficient high chemical stability and weather stability. In addition, the pigments of the prior art show a poor hiding power and no or less metallic colour effect and/or low coloured metallic shine or gloss.

Interference pigments themselves show a poor hiding power. To improve the hiding power absorptive pigments, e. g. carbon black, are added to compensate the hiding power. In certain applications, especially cosmetic formulations (pressed powder, lipsticks, etc.) blends of pigments and Carbon Black may segregate under pressure/shear stress. As a result the optics / cosmetic colour application might look dull and rather black/dirty.

The object of the present invention is to provide pigments which do not show the disadvantages of the pigments of the prior art but show a dark-greyish to black pearlescent effect or a metallic dark blue/green pearlescent effect with high gloss and an increased hiding power. At the same time the pigments should fulfill at least one of the following requirements:
- Pure, neutral dark-grey/dark blue/dark green /dark coloured metallic shades to blackish absorptive colour tone
- No magnetic properties (added by a carbon layer)
- No segregation / no need of additives to prevent segregation
- Adjustable darkness respectively hiding power of the pigment
- Less electrically conductivity.
- No components which are considered to be critical in cosmetic formulations, such as e. g. Prussian Blue, chromium oxide, aluminium
- Increased flowability.

Surprisingly, it has now been found that particles coated with at least one layer of a mixture of amorphous carbon (a-C) and nanocrystalline graphite (nc-graphite) show a dark metallic appearance, a better flowability and at the same time an increased hiding power and an increased UV stability. The optical properties of the coated particles can by influenced by altering the thickness of the a-C/nc-graphite layer.

The present invention relates to pigments according to Claim 1 based on particles, which contain at least one layer, which consists of a mixture of amorphous carbon (a-C) and nanocrystalline graphite (nc-graphite).

The coated particles according to the invention show an improved orientation which is responsible for the metallic appearance, the (liquid) metallic effect and the increased hiding power.

The invention furthermore relates to the use of the pigments according to the invention in paints, coatings, preferably in industrial coatings and automotive coatings, printing inks, security printing inks, plastics, ceramic materials, glasses, as tracer, as filler and in particular in cosmetic formulations and applications and automotive coatings. Furthermore, the pigments according to the invention are also suitable for the preparation of pigment preparations and for the preparation of dry preparations, such as, for example, granules, pearlets, chips, pellets, sausages, briquettes, etc. The dry preparations are used, in particular, in printing inks and in cosmetic formulations.

The dark pigments according to the present invention show a dark metallic or (liquid) metallic or coloured metallic appearance which is very attractive in the final application for example in automotive coatings and cosmetic formulations. Since coatings of a sufficient hiding power are commonly generated by using black iron oxide pigments the pigments according to the present invention might serve as an attractive substitution as these pigment particles are inherently of a non-magnetic and of a less-(heavy) metal nature.

Additionally, the pigments according to the present invention show an improved flowability which is very beneficial for processing and dosing. Furthermore, the pigments show an outstanding dispersibility and no aggregation.

The conformal and homogeneous a-C and nc-graphite layer preferably on top of the pigments' surfaces consists of a mixture of amorphous carbon (a-C) and nanocrystalline graphite (nc-graphite) wherein the weight ratio of a-C : nc-graphite is preferably in the range of 60 : 40 to 80 : 20, in particular of 50 : 50 to 95 : 5 and most particular 80 : 20 to 90 : 10. In a preferred embodiment the a-C/nc-graphite layer contains a higher portion of amorphous carbon compared to the nanocrystalline graphite in the a-C/nc-graphite layer. In a further preferred embodiment, the a-C/nc-graphite layer is precipitated as a final layer on the pigment. However, the a-C/nc-graphite layer can also be an intermediate layer, i.e. deposited between two layers, preferably between two metal oxide layers. The number of a-C/nc-graphite layers is not limited. The layer-arrangement on the surface of a substrate can contain more than a-C/nc-graphite layer, i.e. 1, 2, 3, 4, 5 or even more, but preferably only 1 or 2 layers.

The a-C/nc-graphite layer is preferably prepared by chemical vapor deposition (CVD). The a-C/nc-graphite layer has to be smooth and completely covers the particles with a homogeneous and conformal layer. Compared to the prior art the a-C/nc-graphite layer does not consist of single crystalline carbon domains which are deposited on the surface of a particle but of a layer which is a mixture of amorphous carbon and nanocrystalline graphite and has been grown directly on the particles in the way that a pinhole-free and conformal and homogeneous layer is obtained. The a-C/nc-graphite layer results from heterogenous growth on the surface of particles.

The phase boundaries between a-C and nc-graphite result in a decreased electrical conductivity due to increased transition resistivity at the mentioned phase boundaries. A higher amorphous phase leads to a high number of boundaries which decreases the electrical conductivity. Additionally a-C is intrinsically of a lower electrical conductivity. Consequently the a-C/nc-graphite layer of the present invention shows a low electrical conductive behaviour.

In a preferred embodiment each a-C/nc-graphite layer has a thickness of 0.5 - 10 nm, in particular of 1 - 5 nm, and particularly preferred of 0.5 - 3 nm.

The content of the nanocrystalline graphite based on the particle is very low meaning that the pigments according to the present invention show no or less electrical conductivity.

All known particles which preferably have a particle size of 0.5 - 500 µm or a particle diameter of 1 - 150 µm are suitable as substrate for the pigments according to the present invention. The shape of the particles is not crucial. The particles can be platelet-shaped, needle-shaped, spherical, irregularly shaped. In a preferred embodiment the particles are platelet-shaped or spherical.

The size of the flake-form or platelet shaped particles is not crucial per se and can be matched to the respective application. The flake-form particles have preferably a thickness of 0.05 to 1 µm, in particular 0.1 to 1 µm and very particular preferred of 200 to 500 nm. The size in the other two (lateral) dimensions is usually between 1 and 250 µm, preferably between 2 and 200 µm, and in particular between 5 and 60 µm. It is also possible to employ platelet-shaped particles of different particle sizes. Particular preference is given to a mixture of mica fractions of N mica (10-60 µm), F mica (5-20 µm) and M mica (<15 µm). Preference is furthermore given to N and S fractions (10-130 µm) and F and S fractions (5-130 µm).

Suitable particles are preferably selected from the following group of substrates: natural or synthetic mica, talc, kaolin, Fe₂O₃ flakes, Fe₃O₄ flakes, Al₂O₃ flakes, BiOCI flakes, glass flakes, SiO₂ flakes, TiO₂ flakes, BN flakes, Si-/AI-oxynitride flakes, aluminium flakes, Si-/Ti-Nitride flakes and graphite flakes, pearl essence, synthetic support-free flakes, glass beads, hollow glass beads, silicon pigments, pigments which are based on a substrate, for example filler pigments and effect pigments. Suitable filler pigments and effect pigments are for example interference pigments, multilayer pigments, colour flop pigments, goniochromatic pigments, metal effect pigments, SiO₂ spheres coated with one or more metal oxides, preferably TiO₂ and/or Fe₂O₃.

The particles can be coated with one or more layers, preferably one, two or three layers, in particular with inorganic layers. The inorganic layer preferably comprises absorbent and non-absorbent oxides or hydroxides or metals.

In case the substrate is coated with one or more metal oxide layer(s) and/or metal layer(s) the total layer thickness of all layers on the surface of the substrate is 50-1000 nm, preferably, 100-800 nm, and most preferably 100-500 nm, including the a-C/nc-graphite layer(s). The layer thickness of each a-C/nc-graphite layer has preferably a thickness of 0.5 - 10 nm.

Suitable particles are preferably selected from the following group of substrates: natural or synthetic mica, talc, kaolin, Fe₂O₃ flakes, Fe₃O₄ flakes, Al₂O₃ flakes, BiOCI flakes, glass flakes, SiO₂ flakes, TiO₂ flakes, coated or uncoated SiO₂ spheres, interference pigments based on platelet-shaped substrates and multilayer pigments based on platelet-shaped substrates.

It is also possible to employ mixtures of different particles. Particularly preferred particle mixtures consist of
natural mica flake + SiO₂ flake
natural mica flake + Al₂O₃ flake
natural mica flake + glass flake
natural mica flake + TiO₂ flake
natural mica flake + oxynitride flake
natural mica flake + nitride flake
natural mica flake + pearl essence
natural mica flake + graphite flake
SiO₂ flake + Al₂O₃ flake
glass flake + SiO₂ flake
natural mica flakes + SiO₂ spheres
synthetic mica flakes + SiO₂ spheres
Al₂O₃ flakes + SiO₂ spheres
SiO₂ flakes + SiO₂ spheres
glass flakes + SiO₂ spheres
natural mica flakes + glass spheres
synthetic mica flakes + glass spheres
Al₂O₃ flakes + glass spheres
SiO₂ flakes + glass spheres
glass flakes + glass spheres
synthetic mica flake + SiO₂ flake
synthetic mica flake + Al₂O₃ flake
synthetic mica flake + glass flake
synthetic mica flake + TiO₂ flake
synthetic mica flake + Si-oxynitride flake
synthetic mica flake + Si-/Ti nitride flake
synthetic mica flake + pearl essence
synthetic mica flake + graphite flake
synthetic mica flake + natural mica flake

The particles or the particle mixture are coated with one or more a-C/nc-graphite layer. The a-C/nc-graphite layer can be on the surface and/or can be an intermediate layer in a layer arrangement. The particles are preferably coated on the surface with one a-C/nc-graphite layer.

In a preferred embodiment the particle is an interference pigment or a single-layer or multilayer pigment based on a platelet shaped substrate. Preferred interference pigments are platelet-shaped substrates which are coated with one, two, three or more metal oxide layers. The a-C/nc-graphite layer is deposited on the surface of the interference pigments.

The particles (= interference pigment) are preferably coated with at least one high refractive index layer, like a layer of metal oxide, for example, TiO₂, ZrO₂, SnO₂, ZnO, Ce₂O₃, Fe₂O₃, Fe₃O₄, FeTiOs, Cr₂O₃, CoO, Co₃O₄, VO₂, V₂O₃, NiO, furthermore of titanium suboxides (TiO₂ partially reduced with oxidation states from < 4 to 2, such as the lower oxides Ti₃O₅, Ti₂O₃, TiO), titanium oxynitrides, FeO(OH), thin semitransparent metal layers, for example comprising Al, Fe, Cr, Ag, Au, Pt or Pd, or combinations thereof.

The TiO₂ layer may be in the rutile or anatase modification. In general, the highest quality and gloss and at the same time the most stable pigments are obtained when the TiO₂ is in the rutile modification. In order to obtain the rutile modification, an additive can be used which is able to direct the TiO₂ into the rutile modification. Useful rutile directors are disclosed in the U.S. 4,038,099 and U.S. 5,433,779 and EP 0 271 767. A preferred rutile director is SnO₂.

Preferred particles are coated platelet shaped substrates which contain one or more layers of metal oxides, preferably one metal oxide layer only, in particular selected from TiO₂, Fe₂O₃, Fe₃O₄, SnO₂, ZrO₂ or Cr₂O₃. Especially preferred are natural mica, synthetic mica, glass flakes, SiO₂ flakes and Al₂O₃ flakes which are coated with TiO₂ or Fe₂O₃ and mixtures thereof.

The term "high refractive index" in this patent application means that the refractive index n is ≥ 1.8. The term "low refractive index" in this patent application means that the refractive index n is < 1.8.

The thickness of each high-refractive-index layer depends on the desired interference colour. The thickness of each layer on the surface of the platelet shaped particles is preferably 20-400 nm, preferably 30-300 nm, in particular 30-200 nm.

The number of layers on the surface of the substrates is preferably one or two, furthermore three, four, five, six or seven layers.

In particular, interference packages consisting of high- and low-refractive-index layers on the surface of the platelet shaped substrates result in pigments having increased gloss and a further increased interference colour or colour flop.

Suitable colourless low-refractive-index materials for coating are preferably metal oxides or the corresponding oxide hydrates, such as, for example, SiO₂, Al₂O₃, AIO(OH), B₂O₃, MgO*SiO₂, CaO*SiO₂, Al₂O₃*SiO₂, B₂O₃*SiO₂ compounds such as MgF₂ or a mixture of said metal oxides.

A preferred multilayer system applied on the surface of a platelet shaped substrate is a TiO₂-SiO₂-TiO₂ sequence or TiO₂-MgO*SiO₂-TiO₂ sequence.

The platelet shaped particles can also be coated with one or more layers of a metal or metal alloy selected e.g. from chromium, nickel, silver, bismuth, copper, tin, hastelloy or with a metal sulfide or sulfides selected e.g. of tungsten, molybdenum, cerium, lanthanum or rare earth elements.

The a-C/nc-graphite layer(s) can be deposited directly on the surface of the substrate, between one or more metal or metal oxide layers or deposited on the surface of each metal or metal oxide layer or on the surface of the particle. In a preferred embodiment at least one a-C/nc-graphite layer is applied on the surface of the particles, in particular on the surface of interference pigments and multilayer pigments.

Preferred pigments according to the present invention are mentioned in the following:
substrate + a-C/nc-graphite layer
substrate + a-C/nc-graphite layer + TiO₂
substrate + a-C/nc-graphite layer + Fe₂O₃
substrate + a-C/nc-graphite layer + Fe₃O₄
substrate + a-C/nc-graphite layer + Cr₂O₃
substrate + a-C/nc-graphite layer + SnO₂
substrate + a-C/nc-graphite layer + SiO₂
substrate + a-C/nc-graphite layer + ZrO₂
substrate + a-C/nc-graphite layer + ZnO
substrate + a-C/nc-graphite layer + Al
substrate + a-C/nc-graphite layer + Fe
substrate + a-C/nc-graphite layer + Cr
substrate + TiO₂ + a-C/nc-graphite layer
substrate + TiO₂/Fe₂O₃ + a-C/nc-graphite layer
substrate + Fe₂O₃ + a-C/nc-graphite layer
substrate + Fe₃O₄ + a-C/nc-graphite layer
substrate + TiO₂ + Fe₂O₃ + a-C/nc-graphite layer
substrate + TiO₂ + Fe₃O₄ + a-C/nc-graphite layer
substrate + TiO₂ + SiO₂ + TiO₂ + a-C/nc-graphite layer
substrate + TiO₂ + Al₂O₃ + TiO₂ + a-C/nc-graphite layer
substrate + TiO₂ + MgO*SiO₂ + TiO₂ + a-C/nc-graphite layer
substrate + TiO₂ + CaO*SiO₂ + TiO₂ + a-C/nc-graphite layer
substrate + TiO₂ + Al₂O₃*SiO₂ + TiO₂ + a-C/nc-graphite layer
substrate + TiO₂ + B₂O₃*SiO₂ + TiO₂ + a-C/nc-graphite layer
substrate + Fe₂O₃ + SiO₂ + TiO₂ + a-C/nc-graphite layer
substrate + Fe₂O₃ + Al₂O₃ + TiO₂ + a-C/nc-graphite layer
substrate + Fe₂O₃ + MgO*SiO₂ + TiO₂ + a-C/nc-graphite layer
substrate + Fe₂O₃ + CaO*SiO₂ + TiO₂ + a-C/nc-graphite layer
substrate + Fe₂O₃ + Al₂O₃*SiO₂ + TiO₂ + a-C/nc-graphite layer
substrate + Fe₂O₃ + B₂O₃*SiO₂ + TiO₂ + a-C/nc-graphite layer
substrate + TiO₂/Fe₂O₃ + SiO₂ + TiO₂ + a-C/nc-graphite layer
substrate + TiO₂/Fe₂O₃ + Al₂O₃ + TiO₂ + a-C/nc-graphite layer
substrate + TiO₂/Fe₂O₃ + MgO*SiO₂ + TiO₂ + a-C/nc-graphite layer
substrate + TiO₂/Fe₂O₃ + CaO*SiO₂ + TiO₂+ a-C/nc-graphite layer
substrate + TiO₂/Fe₂O₃ + Al₂O₃*SiO₂ + TiO₂+ a-C/nc-graphite layer
substrate + TiO₂/Fe₂O₃ + B₂O₃*SiO₂ + TiO₂+ a-C/nc-graphite layer
substrate + TiO₂ + SiO₂ + TiO₂/Fe₂O₃ + a-C/nc-graphite layer
substrate + TiO₂/Fe₂O₃ + SiO₂ + TiO₂/Fe₂O₃ + a-C/nc-graphite layer
substrate + TiO₂/Fe₂O₃ + MgO*SiO₂ + TiO₂/Fe₂O₃ + a-C/nc-graphite layer
substrate + TiO₂ + Al₂O₃ + TiO₂/Fe₂O₃+ a-C/nc-graphite layer
substrate+ TiO₂+ MgO*SiO₂ + TiO₂/Fe₂O₃+ a-C/nc-graphite layer
substrate+ TiO₂+ CaO*SiO₂ + TiO₂/Fe₂O₃+ a-C/nc-graphite layer
substrate+ TiO₂ + Al₂O₃*SiO₂ + TiO₂/Fe₂O₃+ a-C/nc-graphite layer
substrate+ TiO₂+ B₂O₃*SiO₂ + TiO₂/Fe₂O₃+ a-C/nc-graphite layer
substrate+ TiO₂ + SiO₂+ a-C/nc-graphite layer
substrate+ TiO₂ + SiO₂/Al₂O₃+ a-C/nc-graphite layer
substrate+ TiO₂ + Al₂O₃+ a-C/nc-graphite layer
substrate+ SnO₂ + a-C/nc-graphite layer
substrate+ SnO₂ + TiO₂ + a-C/nc-graphite layer
substrate+ SnO₂ + Fe₂O₃ + a-C/nc-graphite layer
substrate+ SiO₂ + a-C/nc-graphite layer
substrate+ SiO₂ + TiO₂ + a-C/nc-graphite layer
substrate+ SiO₂ + TiO₂/Fe₂O₃+ a-C/nc-graphite layer
substrate+ SiO₂ + Fe₂O₃ + a-C/nc-graphite layer
substrate+ SiO₂ + TiO₂ + Fe₂O₃ + a-C/nc-graphite layer
substrate+ SiO₂ + TiO₂ + Fe₃O₄ + a-C/nc-graphite layer
substrate+ SiO₂ + TiO₂+ SiO₂ + TiO₂ + a-C/nc-graphite layer
substrate+ SiO₂ + Fe₂O₃ + SiO₂ + TiO₂ + a-C/nc-graphite layer
substrate+ SiO₂ + TiO₂/Fe₂O₃ + SiO₂ + TiO₂ + a-C/nc-graphite layer
substrate+ SiO₂ + TiO₂+ SiO₂ + TiO₂/Fe₂O₃ + a-C/nc-graphite layer
substrate+ SiO₂ + TiO₂ + SiO₂ + a-C/nc-graphite layer
substrate+ SiO₂ + TiO₂ + SiO₂/Al₂O₃ + a-C/nc-graphite layer
substrate+ SiO₂ + TiO₂ + Al₂O₃ + a-C/nc-graphite layer
substrate + a-C/nc-graphite layer + TiO₂ + a-C/nc-graphite layer
substrate + a-C/nc-graphite layer + Fe₂O₃ + a-C/nc-graphite layer
substrate + a-C/nc-graphite layer + SiO₂ + SnO₂ + TiO₂ + a-C/nc-graphite layer
substrate + a-C/nc-graphite layer + SiO₂ + SnO₂ + TiO₂ + a-C/nc-graphite layer + TiO₂
substrate + a-C/nc-graphite layer + SiO₂ + SnO₂ + TiO₂ + a-C/nc-graphite layer + Fe₂O₃

In a particular preferred embodiment the above mentioned preferred pigments are based on platelet-shaped substrates, in particular selected from natural mica and synthetic mica.

The TiO₂ layer(s) in the preferred embodiments mentioned above can be in the rutile or anatase modification.

The synthetic substrates such as synthetic mica, glass flakes, SiO₂ flakes or Al₂O₃ flakes, in the above mentioned preferred embodiments can be doped or undoped. The amount of dopant is preferably in the range of 0.005-5 wt.% based on the substrate.

By the use of one or more a-C/nc-graphite layers it is possible to vary or adjust the colour, lustre and hiding powder of the pigments in a broad range.

In a preferred embodiment the pigments according to the present invention contain only one a-C/nc-graphite layer which is the outer layer applied on the surface of the particle. The particle can also be a substrate like mica, passivated aluminium flakes, glass flakes, etc., which is coated on the surface with an a-C/nc-graphite layer.

The pigments containing a least one carbon/graphite layer show an excellent hiding power and a dark metallic appearance.

The pigments according to the present invention consist preferably of 90 - 99 wt.% particle and 10 - 1 wt.% a-C/nc-graphite layer based on the total pigment.

The coating of the substrates with at least one metal oxide layer preferably takes place by wet chemical coating, by CVD or PVD processes.

The metal-oxide layers on the surface of the substrates are preferably applied by the wet-chemical coating methods developed for the preparation of pearlescent pigments. Methods of this type are described, for example, in US 3087828, US 3087829, US 3553001, DE 14 67 468, DE 19 59 988, DE 20 09 566, DE 22 14 545, DE 22 15 191, DE 22 44 298, DE 23 13 331, DE 25 22 572, DE 31 37 808, DE 31 37 809, DE 31 51 343, DE 31 51 354, DE 31 51 355, DE 32 11 602, DE 32 35 017, DE 196 18 568, EP 0 659 843, or also in further patent documents and other publications known to the person skilled in the art.

In a preferred embodiment the conformal and homogeneous a-C/nc-graphite layer is obtained by a fluidized bed assisted CVD (FBCVD) process which is operated at temperatures ranging from 200 to < 500 °C. The carbon source is selected from carbon containing organic solvents, in particular solvents which decompose at temperatures below 500 °C, such as ethanol, isopropanol, 2-methyl-3-butin-2-ol or sugar compounds such as icing sugar, glucose, fructose, dextrose, or any other sugars known to a person skilled in the art. The carbon precursor can be in liquid or solid form. A mixture of liquid carbon and solid carbon precursor is also possible. It is also possible to use as a carbon precursor a mixture of different organic solvents or a mixture of different sugars or a mixture of sugar and solvent. In a preferred embodiment only one carbon source is used, i.e. a solvent or a solid sugar.

The particle is heated in a fluidized bed reactor upon the desired temperatures ranging from 200 to < 500 °C, preferably 200 to 480 °C and in particular from 250 to 450 °C. The heating and the carbon decomposition reaction takes place in an inert gas atmosphere, for example under N₂, argon, helium. The inert fluidization gas is preferably adjusted in a way that the minimum fluidization velocity of 2 to 6 mm/s, preferably 2 to 4 mm/s, is maintained throughout the process. If the desired reaction temperature is reached the carbon precursors, like organic solvents or sugar compounds, are added to the fluidization gas. After the chemical vapor deposition, the reactor is cooled down under inert gas atmosphere until room temperature is reached. Further post-processing of the obtained pigments might contain sieving depending on the desired application of the pigment.

In particular, the deposition of a thin a-C/nc-graphite layer of at least 4 nm on the coated or uncoated particles enhances the hiding power by a factor from 3.5 to 4.4 compared to the particles which do not contain the a-C/nc-graphite layer. Furthermore, the a-C/nc-graphite layer increases the UV stability of the pigment.

The invention also relates to a process for the preparation of the pigments according to the invention.

The term "coating(s)" or "layer(s)" in this patent application is taken to mean the complete covering/enveloping of the respective surface of the coated or uncoated substrates or particles.

In order to further increase the light, water and weather stability, it is frequently advisable, depending on the area of application, to subject the pigments according to the invention to post-coating or post-treatment. Suitable post-coating or post-treatment methods are, for example, those described in German patent 22 15 191, DE-A 31 51 354, DE-A 32 35 017 or DE-A 33 34 598. This post-coating further increases the chemical and photochemical stability or makes handling of the pigment mixture, in particular incorporation into various media, easier. In order to improve the wettability, dispersibility and/or compatibility with the application media, functional coatings comprising Al₂O₃ or ZrO₂ or mixtures thereof can be applied to the pigment surface. Furthermore, organic post-coatings are possible, for example with silanes, as described, for example, in EP 0090259, EP 0 634 459, WO 99/57204, WO 96/32446, WO 99/57204, US 5,759,255, US 5,571,851, WO 01/92425 or in J.J. Ponjeé, Philips Technical Review, Vol. 44, No. 3, 81 ff. and P.H. Harding, J.C. Berg, J. Adhesion Sci. Technol. Vol. 11 No. 4, pp. 471-493.

The pigments according to the present invention are compatible with a multiplicity of colour systems, preferably from the area of paints, coatings and printing inks. A multiplicity of binders, in particular water-soluble products, as marketed, for example, by BASF, Marabu, Pröll, Sericol, Hartmann, Gebr. Schmidt, Sicpa, Aarberg, Siegwerk, GSB-Wahl, Follmann, Ruco or Coates Screen GmbH, are suitable for the preparation of printing inks for, for example, gravure printing, flexographic printing, offset printing or offset overprint varnishing. The printing inks can be water-based or solvent-based.

It goes without saying that the pigments according to the invention can also advantageously be employed for the various applications as a blend with, for example,
- metal-effect pigments, for example based on iron flakes or aluminium flakes;
- pearlescent pigments based on metal oxide-coated synthetic mica flakes, natural mica flakes, glass flakes, Al₂O₃ flakes, Fe₂O₃ flakes or SiO₂ flakes;
- interference pigments based on metal oxide-coated synthetic mica flakes, natural mica flakes, glass flakes, Al₂O₃ flakes, Fe₂O₃ flakes or SiO₂ flakes;
- goniochromatic pigments;
- multilayered pigments (preferably comprising 2, 3, 4, 5 or 7 layers) based on metal oxide-coated synthetic mica flakes, natural mica flakes, glass flakes, Al₂O₃ flakes, Fe₂O₃ flakes or SiO₂ flakes;
- organic dyes;
- organic pigments;
- inorganic pigments, such as, for example, transparent and opaque white, coloured and black pigments;
- flake-form iron oxides;
- carbon black.

The pigments according to the invention can be mixed in any ratio with commercially available pigments and/or further commercially available fillers.

Commercially available fillers which may be mentioned are, for example, natural and synthetic mica, nylon powder, pure or filled melamine resins, talc, glasses, kaolin, oxides or hydroxides of aluminium, magnesium, calcium, zinc, BiOCI, barium sulfate, calcium sulfate, calcium carbonate, magnesium carbonate, carbon, boron nitride and physical or chemical combinations of these substances. There are no restrictions with respect to the particle shape of the filler. It can be, for example, flake-shaped, spherical or needle-shaped, in accordance with requirements.

The pigments according to the invention can of course also be combined in the formulations with any type of cosmetic raw materials and assistants. These include, inter alia, oils, fats, waxes, film formers, preservatives and assistants which generally determine applicational properties, such as, for example, thickeners and rheological additives, such as, for example, bentonites, hectorites, silicon dioxides, Ca silicates, gelatins, high-molecular-weight carbohydrates and/or surface-active assistants, etc.

The pigments according to the invention are simple and easy to handle. The pigments can be incorporated into the system in which it is used by simple stirring. The pigments according to the present invention show an increased powder flowability which is very beneficial for processing.

The pigments according to the invention can be used for pigmenting coating materials, printing inks, plastics, agricultural films, button pastes, for the coating of seed, for the colouring of food, coatings of medicaments or cosmetic formulations. The concentration of the pigments in the system in which it is to be used for pigmenting is generally between 0.01 and 50 % by weight, preferably between 0.1 and 5 % by weight, based on the overall solids content of the system. This concentration is generally dependent on the specific application.

Plastics containing the pigments according to the invention in amounts of 0.1 to 50 % by weight, in particular from 0.5 to 7 % by weight, are frequently notable for a particular dark metallic and gloss effect.

In the coating sector, especially in automotive coatings and automotive refinishing, the pigments according to the invention are employed in amounts of 0.5 to 10 % by weight. To be used in e.g. an automotive coating the pigments according to the present invention are incorporated into a base coat formulation consisting of a mixture of resins (e.g. polyester, melamine and polyurethane) in combination with amines for pH-adjustment, co-solvents to improve film formation, at least one thickener to adjust rheology. To achieve a sprayable viscosity, defoamer, wetting agents, if necessary further additives, fillers, pigments and/or matting agents and water are added. This base coat is applied on the desired substrate by spray coating. The resulting dry film thickness shall be 10 - 20 µm, preferably 12-18µm. After predrying a clearcoat is applied on top of this basecoat and the complete coating is stoved.

In the coating material, the pigments according to the invention have the advantage that the desired metallic (liquid) colour and gloss is obtained by a single-layer coating (one-coat systems or as a base coat in a two-coat system). In a preferred embodiment the pigments according to the present invention are used in the base coat.

No limits are set for the concentrations of the pigments according to the invention in the cosmetic formulation. They can be - depending on the application - between 0.001 (rinse-off products, for example shower gels) and 60 %. The pigments according to the invention may furthermore also be combined with cosmetic active compounds. Suitable active compounds are, for example, insect repellents, inorganic UV filters, such as, for example, TiO₂, UV A/BC protection filters (for example OMC, B3, MBC), anti-ageing active compounds, vitamins and derivatives thereof (for example vitamin A, C, E, etc.), self-tanning agents (for example DHA, erythrulose, inter alia) and further cosmetic active compounds, such as, for example, bisabolol, LPO, ectoin, emblica, allantoin, bioflavonoids and derivatives thereof.

Organic UV filters are generally employed in an amount of 0.5 - 10 % by weight, preferably 1-8 % by weight, inorganic UV filters in an amount of 0.1 - 30 % by weight, based on the formulation.

In addition, the formulations may comprise further conventional skin-protecting or skin-care active ingredients, such as, for example, aloe vera, avocado oil, coenzyme Q10, green tea extract and also active-compound complexes.

The present invention likewise relates to formulations, in particular cosmetic formulations, which, besides the pigments according to the invention, contain at least one constituent selected from the group of absorbents, astringents, antimicrobial substances, antioxidants, antiperspirants, antifoaming agents, antidandruff active compounds, antistatics, binders, biological additives, bleaches, chelating agents, deodorisers, emollients, emulsifiers, emulsion stabilisers, dyes, humectants, film formers, fillers, fragrances, flavours, insect repellents, preservatives, anticorrosion agents, cosmetic oils, solvents, water, oxidants, vegetable constituents, buffer substances, reducing agents, surfactants, propellant gases, opacifiers, UV filters and UV absorbers, denaturing agents, aloe vera, avocado oil, coenzyme Q10, green tea extract, viscosity regulators, perfume and vitamins.

The invention thus also relates to the use of the pigments according to the present invention in paints, coatings, automobile coatings, automotive finishing, industrial coatings, paints, powder coatings, printing inks, security printing inks, plastics, ceramic materials, cosmetics. The pigments according to the present invention can furthermore be employed in glasses, in paper, in paper coating, in toners for electrophotographic printing processes, in seed, in greenhouse sheeting and tarpaulins, in thermally conductive, self-supporting, electrically insulating, flexible sheets for the insulation of machines or devices, as absorber in the laser marking of paper and plastics, as absorber in the laser welding of plastics, in pigment pastes with water, organic and/or aqueous solvents, in pigment preparations and dry preparations, such as, for example, granules, for example in clear coats in the industrial and automobile sectors, in sunscreens, as filler, in particular in automobile coatings and automotive refinishing and in cosmetic formulations.

All percentage data in this application are per cent by weight, unless indicated otherwise.

The following examples are intended to explain the invention in greater detail, but without restricting it.

### Examples

### Example 1

150 g of natural mica flakes of a particle size from 5 to 15 µm are dispersed in 2000 ml DI water while stirring. The suspension is then heated up until 75 °C while continuous stirring. Precipitation pH-value of suspension is set to 1.8 by adding a SnCl₄ solution (50 %) drop wisely. The remaining SnCl₄ solution is dosed steadily to the suspension. During the procedure the pH value is kept constant at 1.8 by adding sodium hydroxide (32 %). After completing adding the solutions the suspension is stirred for another 10 min.

At a constant pH value of 1.4, 135 g of TiCl₄ solution (25 %) are dosed in until the colour end point (blueish silver) has been reached, i. e. 12 wt.-%, TiO₂. Thus, TiO₂ layer thickness of 12 nm is realized. During the precipitation process pH value is kept constant by continuously adding a 32 % sodium hydroxide solution. After completion the suspension is stirred for another 10 min, filtered off with suction and washed with DI water until salt-fee. The particulate matter is dried at 120 °C for 24 h. After the drying process a calcination step at 800 °C for 45 min follows.

The obtained pigments have an intense blueish to light silvery shade.

### Example 2

150 g of natural mica flakes of a particle size from 5 to 15 µm are dispersed in 2000 ml DI water while stirring. The suspension is then heated up until 75 °C while continuous stirring. Precipitation pH-value of suspension is set to 1.8 by adding a SnCl₄ solution (50 %) drop wisely. The remaining SnCl₄ solution is dosed steadily to the suspension. During the procedure the pH value is kept constant at 1.8 by adding sodium hydroxide (32 %). After completing adding the solutions the suspension is stirred for another 10 min.

At a constant pH value of 1.4, 201 g of TiCl₄ solution (25 %) are dosed in until the colour end point (blueish silver) has been reached, i. e. 18 wt.-% TiO₂. Thus, TiO₂ layer thickness of 18 nm is realized. During the precipitation process pH value is kept constant by continuously adding a 32 % sodium hydroxide solution. After completion the suspension is stirred for another 10 min, filtered off with suction and washed with DI water until salt-fee. The particulate matter is dried at 120 °C for 24 h. After the drying process a calcination step at 800 °C for 45 min follows.

The obtained pigments have a light blueish to intense silvery shade.

### Example 3

150 g of natural mica flakes of a particle size from 5 to 15 µm are dispersed in 2000 ml DI water while stirring. The suspension is then heated up until 75 °C while continuous stirring. Precipitation pH-value of suspension is set to 1.8 by adding a SnCl₄ solution (50 %) drop wisely. The remaining SnCl₄ solution is dosed steadily to the suspension. During the procedure the pH value is kept constant at 1.8 by adding sodium hydroxide (32 %). After completing adding the solutions the suspension is stirred for another 10 min.

At a constant pH value of 1.4, 390 g of TiCl₄ solution (25 %) are dosed in until the colour end point (blueish silver) has been reached, i. e. 35 wt.-% TiO₂. Thus, TiO₂ layer thickness of 35 nm is realized. During the precipitation process pH value is kept constant by continuously adding a 32 % sodium hydroxide solution. After completion the suspension is stirred for another 10 min, filtered off with suction and washed with DI water until salt-fee. The particulate matter is dried at 120 °C for 24 h. After the drying process a calcination step at 800 °C for 45 min follows.

The obtained pigments have a strong silvery shade with light blueish highlights.

### Example 4 - Chemical Vapour Deposition

150 g of the blueish-silvery coloured particles according to Example 1 are heated up in a fluidized bed reactor (DI: 63 mm) up to 490 °C under a constant inert gas atmosphere (N₂). Volumetric flow has been adjusted to reach the minimal fluidization velocity of 2 mm/s, thus excellent mixing and heat and mass transfer properties are guaranteed. As soon as the reaction temperature has been reached the adding of the C precursor acetone is dosed to the fluidization volumetric flow. Due to the elevated reaction temperature the C precursor will decompose in a way that the growth of the C layers on the blueish-silvery coloured pigments surfaces is initiated. The CVD process is run for 60 min in order to achieve a C layer thickness 4 nm. After a cooling phase under inert gas atmosphere the final pigments are removed from the reactor and sieved.

The dark pigments show a metallic effect with high lustre and high hiding power.

The deposited C layer consists of a mixture of a-C and nc-graphite with a weight ratio of 90 :10. The ratio was determined combining RAMAN spectroscopic investigations according to Ferrari et al. and thermogravimetric analysis according to Müller et. al [Müller, J-O; Su, Dang Sheng; Jentoft, Rolf E.; Kröhnert, Jutta; Jentoft, Friederike C.; Schlögl, Robert; Morphology-controlled reactivity of carbonaceous materials towards oxidation, in: Catalysis Today, 102, 2005, S. 259-265.] and Trigueiro et al. [Trigueiro, João Paulo C.; Silva, Glaura G.; Lavall, Rodrigo L.; Furtado, Clascidia A.; Oliveira, Sérgio; Ferlauto, Andre S.; Lacerda, Rodrigo G.; Ladeira, Luiz O.; Liu, Jiang-Wen; Frost, Ray L.; Purity evaluation of carbon nanotube materials by thermogravimetric, TEM, and SEM methods, in: Journal of nanoscience and nanotechnology, 7, 2007, S. 3477-3486.]

### Example 5 - Chemical Vapour Deposition

150 g of the blueish-silvery coloured particles according to Example 2 are heated up in a fluidized bed reactor (DI: 63 mm) up to 490 °C under a constant inert gas atmosphere (N₂). Volumetric flow has been adjusted to reach the minimal fluidization velocity of 2 mm/s, thus excellent mixing and heat and mass transfer properties are guaranteed. As soon as the reaction temperature has been reached the adding of the C precursor 2-methyl-3-butin-2-ol is dosed to the fluidization volumetric flow. Due to the elevated reaction temperature the C precursor will decompose in a way that the growth of the C layers on the particles' surfaces is initiated. The CVD process is run for 60 min in order to achieve a C layer thickness 4 nm. After a cooling phase under inert gas atmosphere the final pigments are removed from the reactor and sieved.

The dark pigments show a deep metallic effect with high lustre and high hiding power.

The deposited C layer consists of a mixture of a-C and nc-graphite with a weight ratio of 90:10. The ratio was determined combining RAMAN spectroscopic investigations according to Ferrari et al. and thermogravimetric analysis according to Müller et al.

### Example 6 - Chemical Vapour Deposition

150 g of the blueish-silvery coloured pigments particles according to Example 3 are heated up in a fluidized bed reactor (DI: 63 mm) up to 490 °C under a constant inert gas atmosphere (N₂). Volumetric flow has been adjusted to reach the minimal fluidization velocity of 2 mm/s, thus excellent mixing and heat and mass transfer properties are guaranteed. As soon as the reaction temperature of 490 °C has been reached the C precursor acetone is dosed to the fluidization flow. Due to the elevated reaction temperature the C precursor will decompose in a way that the growth of the C layers on the particles' surfaces is initiated. The CVD process is run for 60 min in order to achieve a C layer thickness 4 nm. After a cooling phase under inert gas atmosphere (N₂) the final pigments are removed from the reactor and sieved.

The dark pigments show a deep metallic effect, high lustre and high hiding power.

The deposited C layer consists of a mixture of a-C and nc-graphite with a ratio of 90:10. The ratio was determined combining RAMAN spectroscopic investigations according to Ferrari et al. and thermogravimetric analysis according to Müller et al.

### Example 7 - a-C/nc-graphite coating on commercially available blue interference pigments

1 kg of commercially available blue interference pigment

| | |
|---|---|
| Example 7a): | Iriodin^{®} 7225 Ultra Blue (Merck KGaA; natural mica coated with TiO₂, particle size 10 - 60 µm) |
| Example 7b): | Timiron^{®} Splendid Blue (Merck KGaA; multilayer pigment based on natural mica coated with TiO₂ and SiO₂, particle size 10-60 µm) |
| Example 7c): | Pyrisma^{®} Colour Space Blue (Merck KGaA; natural mica coated with TiO₂ and SnO₂, particle size 5-35 µm) |
| Example 7d): | Xirona^{®} Caribbean Blue (Merck KGaA, multilayer pigment based on natural mica coated with TiO₂, SiO₂ and SnO₂, particle size 10-60 µm) |
| Example 7e): | Lumina^{®} Royal Exterior Blue (BASF, natural mica coated with TiO₂, SiO₂ and SnO₂, d₁₀ = 10 µm, d₅₀ = 19 µm, d₉₀ = 34 µm) |
| Example 7f): | Mirage Bright Blue (Eckart, borosilicate glass flakes coated with TiO₂ and SnO₂, particle size 10-70 µm) |
| Example 7g): | SynCrystal Blue (Eckart, synthetic mica (fluorophlogopite coated with TiO₂ and SnO₂, particle size 10-50 µm) |
| Example 7h): | XillaMay (Kuncai, synthetic mica coated with TiO₂ and SnO₂, SiO₂ and Ce₂O₃, particle size 6-30 µm) |

is heated in a fluidized bed reactor (DI: 100 mm) up to the desired reaction temperature of 480 °C The heating and the C deposition reaction are run in an inert gas atmosphere (N₂). The inert fluidization gas is adjusted in a way that the minimum fluidization velocity of 2 mm/s is maintained throughout the process. If the reaction temperature of 480 °C is reached the C precursor acetone or 2-methyl-3-butin-2-ol is added to the fluidization gas. After a cooling phase under inert gas (N₂) atmosphere the final pigments are removed from the reactor and sieved.

The deposited C layer consists of a mixture of a-C/nc-graphite:
Example 7a): a-C/nc-graphite ratio: 85:15, layer thickness: 1-2 nm
Example 7b): a-C/nc-graphite ratio: 85:15, layer thickness: 1-2 nm
Example 7c): a-C/nc-graphite ratio: 95:5, layer thickness: 1-2 nm
Example 7d): a-C/nc-graphite ratio: 90:10, layer thickness: 1-2 nm
Example 7e): a-C/nc-graphite ratio: 95:5, layer thickness: 1-2 nm
Example 7f): a-C/nc-graphite ratio: 95:5, layer thickness: 1-2 nm
Example 7g): a-C/nc-graphite ratio: 90:10, layer thickness: 1-2 nm
Example 7h): a-C/nc-graphite ratio: 85:15, layer thickness: 1-2 nm

The coated pigments of Examples 7a) - 7h) show a (dark) masstone blue shade. At the same time the hiding power is improved significantly compared to the non-coated pigments. Furthermore, the a-C/nc-graphite coated pigments appear more metallic compared to the pristine (= non-coated) pigments.

In case of Example 7d) the a-C/nc-graphite layer enhances the colour travel effect, i.e. a very intense colour travel (= multicolour flop of at least three colours) from blue to violet to green. This effect is highly suitable for cosmetic applications, e. g. eyeshadow, lipgloss, lipsticks and nail polish in such a way that a so-called holographic effect can be seen due to the enhanced colour travel.

### Example 8 - Carbon/Graphite coating on commercially available green interference pigments

1 kg of commercially available interference pigment green

| | |
|---|---|
| Example 8a): | Pyrisma^{®} Colour Space Turquoise (Merck KGaA; natural mica coated with TiO₂, particle size 5-35 µm) |
| Example 8b): | Timiron^{®} Splendid Green (Merck KGaA; multilayer pigment based on natural mica coated with TiO₂ and SiO₂, particle size 10-60 µm) |
| Example | 8c):Xirona^{®} Nordic Sunset (Merck KGaA, SiO₂ flakes coated with SnO₂ and TiO₂, particle size: 5-50 µm) |
| Example 8d): | Mirage Dazzling Green (Eckart, borosilicate glass flakes coated with TiO₂ and SnO₂, particle size 150-200 µm) |
| Example 8e): | Adamas^{®} AE-791K-OP Splendor Green (CQV, Al₂O₃ flakes coated with TiO₂ and SnO₂, d₁₀=5 µm, d₅₀=15-19 µm, d₉₀=30 µm) |

is heated in a fluidized bed reactor (DI: 100 mm) up to the desired reaction temperature of 450 °C The heating and the C deposition reaction are run in an inert gas atmosphere. The inert fluidization gas is adjusted in a way that the minimum fluidization velocity of 2 mm/s is maintained throughout the process. If the reaction temperature, e. g. 450 °C is reached the C precursor acetone or 2-methyl-3-butine-2-ol is added to the fluidization gas. After a cooling phase under inert gas atmosphere (e. g.: N₂ the final pigments are removed from the reactor and sieved.

The deposited C layer consists of a mixture of a-C/nc-graphite:
Example 8a): a-C/nc-graphite ratio: 85:15, layer thickness: 1-2 nm
Example 8b): a-C/nc-graphite ratio: 85:15, layer thickness: 1-2 nm
Example 8c): a-C/nc-graphite ratio: 85:15, layer thickness: 1-2 nm
Example 8d): a-C/nc-graphite ratio: 95:5, layer thickness: 1-2 nm
Example 8e): a-C/nc-graphite ratio: 85:15, layer thickness: 1-2 nm

The coated pigments according to Examples 8a) to 8e) show a (dark) masstone green shade and a significantly improved hiding power. Furthermore, the a-C/nc-graphite coated pigments appear more metallic than the pristine (= non-coated) pigments.

In case of Example 8c) the a-C/nc-graphite layer enhances the colour travel effect and shows a very intense colour travel from silver-green to silver-red to green-gold. This effect can especially be exploited in cosmetic applications, e. g. eyeshadow, lipgloss, lipsticks and nail polish in such a way that a so-called holographic effect can be seen due to the enhanced colour travel.

### Examples 9-15 - a-C/nc-graphite coating on commercially available interference pigments

1 kg of commercially available interference pigments selected from the following table

| **Example #** | **Pigment** | **Composition** | **PSD / µm** | **Parameter** | **C layer thickness** |
|---|---|---|---|---|---|
| 9a) | Iriodin^{®} 120 Luster Satin Merck KGaA | Natural mica + TiO₂ | 5-15 | T = 490 °C, t = 120 min, Precursor: Aceton, | 1 - 2 nm |
| | | | | | 85:15 |
| 9b) | Iriodin^{®} Rutil Fine Satin Merck KGaA | Natural mica + TiO₂ | 5-15 | T = 490 °C, t = 120 min, Precursor: 2-Methyl-3-Butin-2-ol | 1 - 2 nm |
| | | | | | 95:5 |
| 9c) | Timiron^{®} SuperSilk MP-1005 Merck KGaA | Natural mica + TiO₂ | 5-15 | T = 480 °C, t = 120 min, Precursor: 2-Methyl-3-Butin-2-ol | 1 - 2 nm |
| | | | | | 95:5 |
| 9d) | Ronastar^{®} Noble Sparks Merck KGaA | Calcium Sodium Borosilicate + SiO₂ + TiO₃ + | 20 -200 | T = 480 °C, t = 120 min, Precursor: 2-Methyl-3-Butin-2-ol | 1 - 2 nm |
| | | | | | 95:5 |
| 9e) | Xirallic^{®} Crystal Silver T60-10 Merck KGaA | Al₂O₃ flakes + SnO₂ + TiO₂ | 5-30 | T = 490 °C, t = 120 min, Precursor: Aceton | 1 - 2 nm |
| | | | | | 85:15 |
| 9f) | Adamas^{®} A E-901 K-SP Splendor White CVQ | Al₂O₃ + SnO₂ TiO₃ + SiO₂ + Silane | d₁₀ = 5 d₅₀ = 15 - 19 d₉₀ = 30 | T = 490 °C, t = 120 min, Precursor: Aceton | 1 - 2 nm |
| | | | | | 85:15 |
| 9g) | Iriodin^{®} 111 Merck KGaA | Mica + TiO₃ | 5-15 | T = 200 °C, t = 30 min Precursor: Icing Sugar | 1 - 2 nm |
| | | | | | 99:1 |
| 9h) | Timiron^{®} Arctic Silver | Mica + TiO2 + SiO2 | 10-60 | T = 450 °C, t = 120 min, Precursor: Acetone | 1 - 2 nm |
| | | | | | 85:15 |
| 10) | Iriodin^{®} 7215 Ultra Red Merck KGaA | Natural mica + TiO₃ | 10-60 | T = 480 °C, t = 120 min, Precursor: 2-Methyl-3-Butin-2-ol | 1 - 2 nm |
| | | | | | 85:15 |
| 11) | Xirona^{®} Le Rouge Merck KGaA | Silica + Fe₂O₃ | 5-50 | T = 450 °C, t = 60 min, Precursor: Acetone | 1 - 2 nm |
| | | | | | 85:15 |
| 12) | Ronastar^{®} Flaming Lights Merck KGaA | Al₂O₃ + Fe₂O₃ | 5-50 | T = 450 °C, t = 60 min, Precursor: Acetone | 1 - 2 nm |
| | | | | | 85:15 |
| 13) | Ronastar^{®} Aqua Sparks Merck KGaA | Ca-Al Borosilicate + SiO₂ + SnO₂ + TiO₂ | 20 -200 | T = 480 °C, t = 60 min, Precursor: Acetone | 1 - 2 nm |
| | | | | | 85:15 |
| 14) | Xirona^{®} Volcanic Fire Merck KGaA | Mica + TiO₂ + SiO₂ + SnO₂ | 10-60 | T = 480 °C, t = 120 min, Precursor: Acetone | 1 - 2 nm |
| | | | | | 85:15 |
| 15) | Xirona^{®} Moonlight Sparks Merck KGaA | Ca-Al Borosilicate + TiO₂ + SiO₂ + SnO₂ | 20 -200 | T = 480 °C, t = 60 min, Precursor: Acetone | 1 - 2 nm |
| | | | | | 85:15 |

is heated in a fluidized bed reactor (DI: 100 mm) up to the desired reaction temperature. The heating and the C deposition reaction are run in an inert N₂-gas atmosphere. The N₂ inert gas fluidization is adjusted in a way that the minimum fluidization velocity of 2 mm/s is maintained throughout the process. If the reaction temperature is reached the C precursor is added to the fluidization gas. After a cooling phase under inert gas atmosphere (N₂) the final pigments are removed from the reactor and sieved.

The C coating of Examples 9b) and 9c) leads to a liquid metal effect - especially in cosmetic applications, e. g. lipsticks, lipgloss, nailpolish. These three C coated pigments have a Liquid Metal Index of 8.58 (Fop Index = 18.09, Graininess = 2.11). So far, such effects could only be achieved by the use of aluminium flakes which are not allowed to be used in lipgloss, lipsticks and eyeshadows to regulatory constraints.

### Application Examples

### Use Example A1 - Coating

The a-C/nc-graphite coated pigments according to Example 4 are incorporated in a base coat MIPA WBC 000 (MIPA SE, Germany) by stirring in. Depending on the desired colour shade a certain concentration of pigment has to be used. To achieve a full shade of the pigment of Example 4 1 wt.% of pigment on formulation is used. If necessary, the coating is adjusted to spray viscosity of 70-75 mPa · s at 1000 s⁻¹ by dilution with deionized water. The pigmented base coat is applied on black-white metal panels (Metopac T21G, purchased at company Leneta) by spray coating. For application an automated spray application Oerter APL 4.6 with a spray gun DeVilbiss AGMD2616 is used (nozzle 1,4 mm, cap 767c). Spray pressure is 4200 mbar, material feeding is about 110 ml/min, distance between spray gun and substrate is approx. 30 cm. The spray gun moves with 0.45 m/s, three layers with an intermediate flash off time of 30 s between each layer are applied. The resulting dry film thickness is 10 - 20 µm, preferably 11-15 µm. It is also possible to apply only one layer with dry film thickness of 1-3 µm in case the carbon content of the pigment is high enough. After predrying of the pigmented layer at room temperature with air circulation a clearcoat is applied on top of this basecoat and the complete coating is stoved.

### Use Example A2 - Coating

The a-C/nc-graphite coated pigments according to Example 5 are incorporated in a base coat MIPA WBC 000 (MIPA SE, Germany) by stirring in. Depending on the desired colour shade a certain concentration of pigment has to be used. To achieve a full shade of the pigment of Example 4 1 wt.% of pigment on formulation is used. If necessary, the coating is adjusted to spray viscosity of 70-75 mPa · s at 1000 s⁻¹ by dilution with deionized water. The pigmented base coat is applied on black-white metal panels (Metopac T21G, purchased at company Leneta) by spray coating. For application an automated spray application Oerter APL 4.6 with a spray gun DeVilbiss AGMD2616 is used (nozzle 1,4 mm, cap 767c). Spray pressure is 4200 mbar, material feeding is about 110 ml/min, distance between spray gun and substrate is approx. 30 cm. The spray gun moves with 0.45 m/s, three layers with an intermediate flash off time of 30 s between each layer are applied. The resulting dry film thickness is 10 - 20 µm, preferably 11-15 µm. It is also possible to apply only one layer with dry film thickness of 1-3 µm in case the carbon content of the pigment is high enough. After predrying of the pigmented layer at room temperature with air circulation a clearcoat is applied on top of this basecoat and the complete coating is stoved.

### Use Example A3 - Lipstick

| **Ingredients** | | **INCI (CTFA)** | **[wt.%]** |
|---|---|---|---|
| **Phase A** | | | |
| Interference Pigment according to Example 6, | (1) | | 15.00 |
| | | | |

| **Phase B** | | | |
|---|---|---|---|
| Oxynex^{®} K liquid | (1) | PEG-8, TOCOPHEROL, ASCORBYL PALMITATE, ASCORBIC ACID, CITRIC ACID | 0.05 |
| Sensiva^{®} PA 20 | (2) | PHENETHYL ALCOHOL, ETHYLHEXYL GLYCERIN | 1.00 |
| Paraffin viscous | (1) | PARAFFINUM LIQUIDUM (MINERAL OIL) | 2.10 |
| Adeps Lanae | (3) | LANOLIN | 3.50 |
| Paracera C 44 | (4) | COPERNICIA CERIFERA CERA (COPERNICIA CERIFERA (CARNAUBA) WAX), CERESIN | 5.25 |
| Isopropyl Myristate | (5) | ISOPROPYL MYRISTATE | 5.60 |
| Wax white | (1) | CERA ALBA (BEESWAX) | 8.75 |
| Castor Oil | (3) | RICINUS COMMUNIS SEED OIL | 58.55 |
| | | | |

| **Phase C** | | | |
|---|---|---|---|
| Fragrance Pearl FEMA | (6) | PARFUM | 0.20 |

### Procedure:

Heat the ingredients of phase B up to 75 °C and stir until completely melted. Add phase A and stir until all ingredients are evenly dispersed. Cool down to 65 °C, stir until the phase is air free, add phase C and pour into lipstick molds preheated to 55 °C. Store the molds in a freezer for approx. 1 hour, remove the sticks and insert them into lipstick mechanics. Flame the lipsticks carefully.

### Suppliers:

| | | | |
|---|---|---|---|
| (2) | Schülke & Mayr GmbH | | |
| (3) | Henry Lamotte Oils GmbH | (4) | Azelis Germany GmbH |
| (5) | BASF AG | (6) | Cosnaderm GmbH |

### Use Example A4 - Lipstick

| **Ingredients** | **INCI (CTFA)** | | **[wt.%]** |
|---|---|---|---|
| **Phase A** | | | |
| Dark Green Interference Pigment according to Example 8b | (1) | | 15.00 |
| | | | |

| **Phase B** | | | |
|---|---|---|---|
| Oxynex^{®} K liquid | (1) | PEG-8, TOCOPHEROL, ASCORBYL PALMITATE, ASCORBIC ACID, CITRIC ACID | 0.05 |
| Sensiva^{®} PA 20 | (2) | PHENETHYL ALCOHOL, ETHYLHEXYL GLYCERIN | 1.00 |
| Paraffin viscous | (1) | PARAFFINUM LIQUIDUM (MINERAL OIL) | 2.10 |
| Adeps Lanae | (3) | LANOLIN | 3.50 |
| Paracera C 44 | (4) | COPERNICIA CERIFERA CERA (COPERNICIA CERIFERA (CARNAUBA) WAX), CERESIN | 5.25 |
| Isopropyl Myristate | (5) | ISOPROPYL MYRISTATE | 5.60 |
| Wax white | (1) | CERA ALBA (BEESWAX) | 8.75 |
| Castor Oil | (3) | RICINUS COMMUNIS SEED OIL | 58.55 |
| | | | |

| **Phase C** | | | |
|---|---|---|---|
| Fragrance Pearl FEMA | (6) | PARFUM | 0.20 |

### Procedure:

Heat the ingredients of phase B up to 75 °C and stir until completely melted. Add phase A and stir until all ingredients are evenly dispersed. Cool down to 65 °C, stir until the phase is air free, add phase C and pour into lipstick molds preheated to 55 °C. Store the molds in a freezer for approx. 1 hour, remove the sticks and insert them into lipstick mechanics. Flame the lipsticks carefully.

### Suppliers:

| | | | |
|---|---|---|---|
| (2) | Schülke & Mayr GmbH | | |
| (3) | Henry Lamotte Oils GmbH | (4) | Azelis Germany GmbH |
| (5) | BASF AG | (6) | Cosnaderm GmbH |

### Use Example A5 - Lipstick

| **Ingredients** | **INCI (CTFA)** | | **[wt%]** |
|---|---|---|---|
| **Phase A** | | | |
| Dark Green Interference Pigment according to Example 8a) | (1) | | 15.00 |
| | | | |

| **Phase B** | | | |
|---|---|---|---|
| Oxynex^{®} K liquid | (1) | PEG-8, TOCOPHEROL, ASCORBYL PALMITATE, ASCORBIC ACID, CITRIC ACID | 0.05 |
| Sensiva^{®} PA 20 | (2) | PHENETHYL ALCOHOL, ETHYLHEXYL GLYCERIN | 1.00 |
| Paraffin viscous | (1) | PARAFFINUM LIQUIDUM (MINERAL OIL) | 2.10 |
| Adeps Lanae | (3) | LANOLIN | 3.50 |
| Paracera C 44 | (4) | COPERNICIA CERIFERA CERA (COPERNICIA CERIFERA (CARNAUBA) WAX), CERESIN | 5.25 |
| Isopropyl Myristate | (5) | ISOPROPYL MYRISTATE | 5.60 |
| Wax white | (1) | CERA ALBA (BEESWAX) | 8.75 |
| Castor Oil | (3) | RICINUS COMMUNIS SEED OIL | 58.55 |
| | | | |

| **Phase C** | | | |
|---|---|---|---|
| Fragrance Pearl FEMA | (6) | PARFUM | 0.20 |

### Procedure:

Heat the ingredients of phase B up to 75 °C and stir until completely melted. Add phase A and stir until all ingredients are evenly dispersed. Cool down to 65 °C, stir until the phase is air free, add phase C and pour into lipstick molds preheated to 55 °C. Store the molds in a freezer for approx. 1 hour, remove the sticks and insert them into lipstick mechanics. Flame the lipsticks carefully.

### Suppliers:

| | | | |
|---|---|---|---|
| (2) | Schülke & Mayr GmbH | | |
| (3) | Henry Lamotte Oils GmbH | (4) | Azelis Germany GmbH |
| (5) | BASF AG | (6) | Cosnaderm GmbH |

### Use Example A6 - Lipstick

| | | | |
|---|---|---|---|
| **Ingredients** | **INCI (CTFA)** | | **[wt%]** |

| **Phase A** | | | |
|---|---|---|---|
| Dark Blue Interference Pigment according to Example 7b) | (1) | | 15.00 |
| | | | |

| **Phase B** | | | |
|---|---|---|---|
| Oxynex^{®} K liquid | (1) | PEG-8, TOCOPHEROL, ASCORBYL PALMITATE, ASCORBIC ACID, CITRIC ACID | 0.05 |
| Sensiva^{®} PA 20 | (2) | PHENETHYL ALCOHOL, ETHYLHEXYL GLYCERIN | 1.00 |
| Paraffin viscous | (1) | PARAFFINUM LIQUIDUM (MINERAL OIL) | 2.10 |
| Adeps Lanae | (3) | LANOLIN | 3.50 |
| Paracera C 44 | (4) | COPERNICIA CERIFERA CERA (COPERNICIA CERIFERA (CARNAUBA) WAX), CERESIN | 5.25 |
| Isopropyl Myristate | (5) | ISOPROPYL MYRISTATE | 5.60 |
| Wax white | (1) | CERA ALBA (BEESWAX) | 8.75 |
| Castor Oil | (3) | RICINUS COMMUNIS SEED OIL | 58.55 |
| | | | |

| **Phase C** | | | |
|---|---|---|---|
| Fragrance Pearl FEMA | (6) | PARFUM | 0.20 |

### Procedure:

Heat the ingredients of phase B up to 75 °C and stir until completely melted. Add phase A and stir until all ingredients are evenly dispersed. Cool down to 65 °C, stir until the phase is air free, add phase C and pour into lipstick molds preheated to 55 °C. Store the molds in a freezer for approx. 1 hour, remove the sticks and insert them into lipstick mechanics. Flame the lipsticks carefully.

### Suppliers:

| | |
|---|---|
| (2) Schülke & Mayr GmbH | |
| (3) Henry Lamotte Oils GmbH | (4) Azelis Germany GmbH |
| (5) BASF AG | (6) Cosnaderm GmbH |

### Use Example A7 - Eye Shadow

| **Ingredients** | | **INCI (CTFA)** | **[wt%]** |
|---|---|---|---|
| **Phase A** | | | |
| Interference Pigment according to Example 6 | (1) | | 30.00 |
| Parteck^{®} LUB Talc | (1) | TALC | 10.00 |
| | | | |

| **Phase B** | | | |
|---|---|---|---|
| RonaCare^{®} AP | (1) | BIS-ETHYLHEXYL HYDROXYDIMETHOXY BENZYLMALONATE | 0.50 |
| Oxynex^{®} K liquid | (1) | PEG-8, TOCOPHEROL, ASCORBYL PALMITATE, ASCORBIC ACID, CITRIC ACID | 0.10 |
| all-rac-alpha-Tocopheryl acetate | (1) | TOCOPHERYL ACETATE | 0.50 |
| Parteck^{®} LUB STA 50 | (1) | STEARIC ACID | 3.00 |
| SP Crodamol PMP MBAL-LQ- (MH) | (2) | PPG-2 MYRISTYL ETHER PROPIONATE | 30.90 |
| Syncrowax HGLC | (2) | C18-36 ACID TRIGLYCERIDE | 10.00 |
| Miglyol^{®} 812 N | (3) | CAPRYLIC/CAPRIC TRIGLYCERIDE | 8.00 |
| Syncrowax HRC | (2) | TRIBEHENIN | 3.00 |
| Ganex^{™} V-216 | (4) | PVP/HEXADECENE COPOLYMER | 2.00 |
| Sunflower Oil, refined | (5) | HELIANTHUS ANNUUS SEED OIL (HELIANTHUS ANNUUS (SUNFLOWER) SEED OIL) | 1.00 |
| Sensiva^{®} PA 20 | (6) | PHENETHYL ALCOHOL, ETHYLHEXYL GLYCERIN | 1.00 |

### Procedure:

Heat phase B to 80 °C until all ingredients are melted. Cool down to 65°C and add the ingredients of phase A while stirring. Fill the bulk into the desired packaging at 65 °C. Cool down to room temperature.

### Suppliers:

| | |
|---|---|
| (2) Croda | |
| (3) IOI Oleo GmbH | (4) Ashland |
| (5) Gustav Heess GmbH | (6) Schülke & Mayr GmbH |

### Use Example A8 - Lip balm

| **Ingredients** | | **INCI (CTFA)** | **[wt%]** |
|---|---|---|---|
| **Phase A** | | | |
| Metallic reddish/brownish interference pigments according to Example 11 | (1) | | 15.00 |
| | | | |

| **Phase B** | | | |
|---|---|---|---|
| Oxynex^{®} K liquid | (1) | PEG-8, TOCOPHEROL, ASCORBYL PALMITATE, ASCORBIC ACID, CITRIC ACID | 0.05 |
| Sensiva^{®} PA 20 | (2) | PHENETHYL ALCOHOL, ETHYLHEXYL GLYCERIN | 1.00 |
| Paraffin viscous | (1) | PARAFFINUM LIQUIDUM (MINERAL OIL) | 2.10 |
| Adeps Lanae | (3) | LANOLIN | 3.50 |
| Paracera C 44 | (4) | COPERNICIA CERIFERA CERA (COPERNICIA CERIFERA (CARNAUBA) WAX), CERESIN | 5.25 |
| Isopropyl Myristate | (5) | ISOPROPYL MYRISTATE | 5.60 |
| Wax white | (1) | CERA ALBA (BEESWAX) | 8.75 |
| Castor Oil | (3) | RICINUS COMMUNIS SEED OIL | 58.55 |
| | | | |

| **Phase C** | | | |
|---|---|---|---|
| Fragrance Pearl FEMA | (6) | PARFUM | 0.20 |

### Procedure:

Heat the ingredients of phase B up to 75 °C and stir until completely melted. Add phase A and stir until all ingredients are evenly dispersed. Cool down to 65 °C, stir until the phase is air free, add phase C and pour into molds. Store the molds in a freezer for approx. 1 hour. The lipstick base is poured into eye shadow pans.

### Suppliers:

| | |
|---|---|
| (2) Schülke & Mayr GmbH | |
| (3) Henry Lamotte Oils GmbH | (4) Azelis Germany GmbH |
| (5) BASF AG | (6) Cosnaderm GmbH |

### Use Example A9 - Lip balm

| **Ingredients** | | **INCI (CTFA)** | **[wt%]** |
|---|---|---|---|
| **Phase A** | | | |
| Metallic reddish/brownish interference pigments according to Example 12 | (1) | | 15.00 |
| | | | |

| **Phase B** | | | |
|---|---|---|---|
| Oxynex^{®} K liquid | (1) | PEG-8, TOCOPHEROL, ASCORBYL PALMITATE, ASCORBIC ACID, CITRIC ACID | 0.05 |
| Sensiva^{®} PA 20 | (2) | PHENETHYL ALCOHOL, ETHYLHEXYL GLYCERIN | 1.00 |
| Paraffin viscous | (1) | PARAFFINUM LIQUIDUM (MINERAL OIL) | 2.10 |
| Adeps Lanae | (3) | LANOLIN | 3.50 |
| Paracera C 44 | (4) | COPERNICIA CERIFERA CERA (COPERNICIA CERIFERA (CARNAUBA) WAX), CERESIN | 5.25 |
| Isopropyl Myristate | (5) | ISOPROPYL MYRISTATE | 5.60 |
| Wax white | (1) | CERA ALBA (BEESWAX) | 8.75 |
| Castor Oil | (3) | RICINUS COMMUNIS SEED OIL | 58.55 |
| | | | |

| **Phase C** | | | |
|---|---|---|---|
| Fragrance Pearl FEMA | (6) | PARFUM | 0.20 |

### Procedure:

Heat the ingredients of phase B up to 75 °C and stir until completely melted. Add phase A and stir until all ingredients are evenly dispersed. Cool down to 65 °C, stir until the phase is air free, add phase C and pour into molds. Store the molds in a freezer for approx. 1 hour. The lipstick base is poured into eye shadow pans.

### Suppliers:

| | |
|---|---|
| (2) Schülke & Mayr GmbH | |
| (3) Henry Lamotte Oils GmbH | (4) Azelis Germany GmbH |
| (5) BASF AG | (6) Cosnaderm GmbH |

## Claims

1. Pigments based on particles **characterized in that** the particles are coated with at least one layer which consists of a mixture of amorphous carbon (a-C) and nanocrystalline graphite (nc-graphite) whereas coated means the complete covering/enveloping of the respective surface of the particles.

2. Pigments according to Claim 1 **characterized in that** the ratio a-C/nc-graphite is in the range of 60 : 40 to 80 : 20.

3. Pigments according to Claim 1 or 2 **characterized in that** the a-C/nc-graphite layer has a thickness of 1 - 10 nm.

4. Pigments according to one or more of Claims 1 to 3 **characterized in that** the particles are selected from the following group of substrates: natural or synthetic mica, talc, kaolin, Fe₂O₃ flakes, Fe₃O₄ flakes, Al₂O₃ flakes, BiOCI flakes, glass flakes, SiO₂ flakes, TiO₂ flakes, BN flakes, aluminum flakes, Si-oxynitride flakes, Si-/Ti-nitride flakes and graphite flakes, pearl essence, synthetic support-free flakes, glass beads, filler pigments, interference pigments, multilayer pigments, colour flop pigments, goniochromatic pigments, metal effect pigments, silicon particles or mixtures thereof.

5. Pigments according to one or more of Claims 1 to 4 **characterized in that** the substrates are spherical or platelet-shaped.

6. Pigments according to one or more of Claims 1 to 5 **characterized in that** the substrates are coated with a least one metal oxide and/or metal.

7. Pigments according to one or more of Claims 1 to 6 **characterized in that** the particles are selected from the following group:
substrate+ a-C/nc-graphite layer
substrate+ a-C/nc-graphite layer + TiO₂
substrate+ a-C/nc-graphite layer + Fe₂O₃
substrate+ a-C/nc-graphite layer + Fe₃O₄
substrate+ a-C/nc-graphite layer + Cr₂O₃
substrate + a-C/nc-graphite layer + SiO₂
substrate + a-C/nc-graphite layer + ZrO₂
substrate+ a-C/nc-graphite layer + SnO₂
substrate+ a-C/nc-graphite layer + ZnO
substrate+ a-C/nc-graphite layer + Al
substrate+ a-C/nc-graphite layer + Fe
substrate+ a-C/nc-graphite layer + Cr
substrate+ TiO₂ + a-C/nc-graphite layer
substrate+ TiO₂/Fe₂O₃ + a-C/nc-graphite layer
substrate+ Fe₂O₃ + a-C/nc-graphite layer
substrate+ Fe₃O₄ + a-C/nc-graphite layer
substrate+ TiO₂ + Fe₂O₃ + a-C/nc-graphite layer
substrate+ TiO₂ + Fe₃O₄ + a-C/nc-graphite layer
substrate+ TiO₂ + SiO₂ + TiO₂ + a-C/nc-graphite layer
substrate+ TiO₂ + Al₂O₃ + TiO₂ + a-C/nc-graphite layer
substrate+ TiO₂ + MgO*SiO₂ + TiO₂ + a-C/nc-graphite layer
substrate+ TiO₂ + CaO*SiO₂ + TiO₂ + a-C/nc-graphite layer
substrate+ TiO₂ + Al₂O₃*SiO₂ + TiO₂ + a-C/nc-graphite layer
substrate+ TiO₂ + B₂O₃*SiO₂ + TiO₂ + a-C/nc-graphite layer
substrate+ Fe₂O₃ + SiO₂ + TiO₂ + a-C/nc-graphite layer
substrate+ Fe₂O₃ + Al₂O₃ + TiO₂ + a-C/nc-graphite layer
substrate+ Fe₂O₃ + MgO*SiO₂ + TiO₂ + a-C/nc-graphite layer
substrate+ Fe₂O₃ + CaO*SiO₂ + TiO₂ + a-C/nc-graphite layer
substrate+ Fe₂O₃ + Al₂O₃*SiO₂ + TiO₂ + a-C/nc-graphite layer
substrate+ Fe₂O₃ + B₂O₃*SiO₂ + TiO₂ + a-C/nc-graphite layer
substrate+ TiO₂/Fe₂O₃ + SiO₂ + TiO₂ + a-C/nc-graphite layer
substrate+ TiO₂/Fe₂O₃ + Al₂O₃ + TiO₂ + a-C/nc-graphite layer
substrate+ TiO₂/Fe₂O₃ + MgO*SiO₂ + TiO₂ + a-C/nc-graphite layer
substrate+ TiO₂/Fe₂O₃ + CaO*SiO₂ + TiO₂+ a-C/nc-graphite layer
substrate+ TiO₂/Fe₂O₃ + Al₂O₃*SiO₂ + TiO₂+ a-C/nc-graphite layer
substrate+ TiO₂/Fe₂O₃ + B₂O₃*SiO₂ + TiO₂+ a-C/nc-graphite layer
substrate+ TiO₂ + SiO₂ + TiO₂/Fe₂O₃ + a-C/nc-graphite layer
substrate + TiO₂/Fe₂O₃ + SiO₂ + TiO₂/Fe₂O₃ + a-C/nc-graphite layer
substrate + TiO₂/Fe₂O₃ + MgO*SiO₂ + TiO₂/Fe₂O₃ + a-C/nc-graphite layer
substrate+ TiO₂ + Al₂O₃ + TiO₂/Fe₂O₃+ a-C/nc-graphite layer
substrate+ TiO₂+ MgO*SiO₂ + TiO₂/Fe₂O₃+ a-C/nc-graphite layer
substrate+ TiO₂+ CaO*SiO₂ + TiO₂/Fe₂O₃+ a-C/nc-graphite layer
substrate+ TiO₂ + Al₂O₃*SiO₂ + TiO₂/Fe₂O₃+ a-C/nc-graphite layer
substrate+ TiO₂+ B₂O₃*SiO₂ + TiO₂/Fe₂O₃+ a-C/nc-graphite layer
substrate+ TiO₂ + SiO₂+ a-C/nc-graphite layer
substrate+ TiO₂ + SiO₂/Al₂O₃+ a-C/nc-graphite layer
substrate+ TiO₂ + Al₂O₃+ a-C/nc-graphite layer
substrate+ SnO₂ + a-C/nc-graphite layer
substrate+ SnO₂ + TiO₂ + a-C/nc-graphite layer
substrate+ SnO₂ + Fe₂O₃ + a-C/nc-graphite layer
substrate+ SiO₂ + a-C/nc-graphite layer
substrate+ SiO₂ + TiO₂ + a-C/nc-graphite layer
substrate+ SiO₂ + TiO₂/Fe₂O₃ + a-C/nc-graphite layer
substrate+ SiO₂ + Fe₂O₃ + a-C/nc-graphite layer
substrate+ SiO₂ + TiO₂ + Fe₂O₃ + a-C/nc-graphite layer
substrate+ SiO₂ + TiO₂ + Fe₃O₄ + a-C/nc-graphite layer
substrate+ SiO₂ + TiO₂ + SiO₂ + TiO₂ + a-C/nc-graphite layer
substrate+ SiO₂ + Fe₂O₃ + SiO₂ + TiO₂ + a-C/nc-graphite layer
substrate+ SiO₂ + TiO₂/Fe₂O₃ + SiO₂ + TiO₂ + a-C/nc-graphite layer
substrate+ SiO₂ + TiO₂ + SiO₂ + TiO₂/Fe₂O₃ + a-C/nc-graphite layer
substrate+ SiO₂ + TiO₂ + SiO₂ + a-C/nc-graphite layer
substrate+ SiO₂ + TiO₂ + SiO₂/Al₂O₃ + a-C/nc-graphite layer
substrate+ SiO₂ + TiO₂ + Al₂O₃ + a-C/nc-graphite layer
substrate + a-C/nc-graphite layer + TiO₂ + a-C/nc-graphite layer
substrate + a-C/nc-graphite layer + Fe₂O₃ + a-C/nc-graphite layer
substrate + a-C/nc-graphite layer + SiO₂ + SnO₂ + TiO₂ + a-C/nc-
graphite layer
substrate + a-C/nc-graphite layer + SiO₂ + SnO₂ + TiO₂ + a-C/nc-graphite layer + TiO₂
substrate + a-C/nc-graphite layer + SiO₂ + SnO₂ + TiO₂ + a-C/nc-graphite layer + Fe₂O₃

8. Pigments according to one or more of Claims 1 to 7 **characterized in that** the pigments consist of 90 - 99 wt.% of particle and 1 - 10 wt.% of a-C/nc-graphite layer(s) based on the total pigment.

9. Process for the preparation of the pigments according to one or more of Claims 1 to 8 **characterized in that** particles are heated in a fluidized bed reactor upon the desired temperature in an inert gas atmosphere and when the desired reaction temperature is reached carbon precursors are added to the fluidization gas and that after the chemical deposition the fluidized bed reaction will be cooled down under inert gas atmosphere until room temperature is reached.

10. Process according to Claim 9, **characterized in that** the carbon precursors are selected from the group of sugars and organic solvents.

11. Process according to Claims 10 and 11 **characterized in that** the precursors are ethanol, isopropanol, acetone, 2-methyl-3-butin-2-ol, icing sugar, fructose, glycose, dextrose.

12. Process according to one or more of claims 10 to 12 **characterized in that** the reaction temperature is 200 to < 500 °C.

13. Process according to one or more of claims 10 to 13 **characterized in that** the fluidized bed reactor is a fluidized bed assisted CVD reactor (FBCVD).

14. Use of the pigments according to one or more of Claims 1 to 8 in paints, coatings, automobile coatings, automotive refinishing, industrial coatings, paints, powder coatings, printing inks, security printing inks, plastics, ceramic materials, cosmetics, glasses, paper, paper coating, toners for electrophotographic printing processes, seeds, greenhouse sheeting and tarpaulins, thermally conductive, self-supporting, electrically insulating, flexible sheets for the insulation of machines or devices, as absorber in the laser marking of paper and plastics, absorber in the laser welding of plastics, pigment pastes with water, organic and/or aqueous solvents, in pigment preparations and dry preparations.

15. Formulation containing pigments according to one or more of Claims 1 to 8 in amounts of 0.01 - 95 % by weight, based on the formulation as a whole.

16. Formulation according to Claim 15 **characterized in that** it additionally contains at least one component selected from the group of absorbents, astringents, antimicrobial substances, antioxidants, antiperspirants, antifoaming agents, antidandruff active compounds, antistatics, binders, biological additives, bleaches, chelating agents, deodorisers, emollients, emulsifiers, emulsion stabilisers, dyes, humectants, film formers, fillers, fragrances, flavours, insect repellents, preservatives, anticorrosion agents, cosmetic oils, solvents, water, oxidants, vegetable constituents, buffer substances, reducing agents, surfactants, propellant gases, opacifiers, UV filters and UV absorbers, denaturing agents, aloe vera, avocado oil, coenzyme Q10, green tea extract, viscosity regulators, perfume and vitamins.

## Patentansprüche

1. Pigmente basierend auf Partikeln, **dadurch gekennzeichnet, dass** die Partikel mit mindestens einer Schicht beschichtet sind, die aus einer Mischung von amorphem Kohlenstoff (a-C) und nanokristallinem Graphit (nc-Graphit) besteht, während beschichtet die vollständige Bedeckung/Umhüllung der jeweiligen Oberfläche der Partikel bedeutet.

2. Pigmente nach Anspruch 1, **dadurch gekennzeichnet, dass** das Verhältnis a-C/nc-Graphit im Bereich von 60 : 40 bis 80 : 20 liegt.

3. Pigmente nach Anspruch 1 oder 2, **dadurch gekennzeichnet, dass** die a-C/nc-Graphit-Schicht eine Dicke von 1 - 10 nm aufweist.

4. Pigmente nach einem oder mehreren der Ansprüche 1 bis 3, **dadurch gekennzeichnet, dass** die Partikel ausgewählt sind aus der folgenden Gruppe von Substraten: natürlicher oder synthetischer Glimmer, Talkum, Kaolin, Fe₂O₃-Plättchen, Fe₃O₄-Plättchen, Al₂O₃-Plättchen, BiOCI-Plättchen, Glasplättchen, SiO₂-Plättchen, TiO₂-Plättchen, BN-Plättchen, Aluminiumplättchen, Si-Oxynitrid-Plättchen, Si-/Ti-Nitrid-Plättchen und Graphitplättchen, Fischsilber, synthetische trägerfreie Plättchen, Glasperlen, Füllpigmente, Interferenzpigmente, Mehrschichtpigmente, Farbflop-Pigmente, goniochromatische Pigmente, Metalleffektpigmente, Siliziumpartikel oder Mischungen davon.

5. Pigmente nach einem oder mehreren der Ansprüche 1 bis 4, **dadurch gekennzeichnet, dass** die Substrate kugel- oder plättchenförmig sind.

6. Pigmente nach einem oder mehreren der Ansprüche 1 bis 5, **dadurch gekennzeichnet, dass** die Substrate mit mindestens einem Metalloxid und/oder Metall beschichtet sind.

7. Pigmente nach einem oder mehreren der Ansprüche 1 bis 6, **dadurch gekennzeichnet, dass** die Partikel ausgewählt sind aus der folgenden Gruppe:
Substrat+ a-C/nc-Graphitschicht
Substrat+ a-C/nc-Graphitschicht + TiO₂
Substrat+ a-C/nc-Graphitschicht + Fe₂O₃
Substrat+ a-C/nc-Graphitschicht + Fe₃O₄
Substrat+ a-C/nc-Graphitschicht + Cr₂O₃
Substrat + a-C/nc-Graphitschicht + SiO₂
Substrat + a-C/nc-Graphitschicht + ZrO₂
Substrat+ a-C/nc-Graphitschicht + SnO₂
Substrat+ a-C/nc-Graphitschicht + ZnO
Substrat+ a-C/nc-Graphitschicht + Al
Substrat+ a-C/nc-Graphitschicht + Fe
Substrat+ a-C/nc-Graphitschicht + Cr
Substrat+ TiO₂ + a-C/nc-Graphitschicht
Substrat+ TiO₂/Fe₂O₃ + a-C/nc-Graphitschicht
Substrat+ Fe₂O₃ + a-C/nc-Graphitschicht
Substrat+ Fe₃O₄ + a-C/nc-Graphitschicht
Substrat+ TiO₂ + Fe₂O₃ + a-C/nc-Graphitschicht
Substrat+ TiO₂ + Fe₃O₄ + a-C/nc-Graphitschicht
Substrat+ TiO₂ + SiO₂ + TiO₂ + a-C/nc-Graphitschicht
Substrat+ TiO₂ + Al₂O₃ + TiO₂ + a-C/nc-Graphitschicht
Substrat+ TiO₂ + MgO*SiO₂ + TiO₂ + a-C/nc-Graphitschicht
Substrat+ TiO₂ + CaO*SiO₂ + TiO₂ + a-C/nc-Graphitschicht
Substrat+ TiO₂ + Al₂O₃*SiO₂ + TiO₂ + a-C/nc-Graphitschicht
Substrat+ TiO₂ + B₂O₃*SiO₂ + TiO₂ + a-C/nc-Graphitschicht
Substrat+ Fe₂O₃ + SiO₂ + TiO₂ + a-C/nc-Graphitschicht
Substrat+ Fe₂O₃ + Al₂O₃ + TiO₂ + a-C/nc-Graphitschicht
Substrat+ Fe₂O₃ + MgO*SiO₂ + TiO₂ + a-C/nc-Graphitschicht
Substrat+ Fe₂O₃ + CaO*SiO₂ + TiO₂ + a-C/nc-Graphitschicht
Substrat+ Fe₂O₃ + Al₂O₃*SiO₂ + TiO₂ + a-C/nc-Graphitschicht
Substrat+ Fe₂O₃ + B₂O₃*SiO₂ + TiO₂ + a-C/nc-Graphitschicht
Substrat+ TiO₂/Fe₂O₃ + SiO₂ + TiO₂ + a-C/nc-Graphitschicht
Substrat+ TiO₂/Fe₂O₃ + Al₂O₃ + TiO₂ + a-C/nc-Graphitschicht
Substrat+ TiO₂/Fe₂O₃ + MgO*SiO₂ + TiO₂ + a-C/nc-Graphitschicht
Substrat+ TiO₂/Fe₂O₃ + CaO*SiO₂ + TiO₂+ a-C/nc-Graphitschicht
Substrat+ TiO₂/Fe₂O₃ + Al₂O₃*SiO₂ + TiO₂+ a-C/nc-Graphitschicht
Substrat+ TiO₂/Fe₂O₃ + B₂O₃*SiO₂ + TiO₂+ a-C/nc-Graphitschicht
Substrat+ TiO₂ + SiO₂ + TiO₂/Fe₂O₃ + a-C/nc-Graphitschicht
Substrat + TiO₂/Fe₂O₃ + SiO₂ + TiO₂/Fe₂O₃ + a-C/nc-Graphitschicht
Substrat + TiO₂/Fe₂O₃ + MgO*SiO₂ + TiO₂/Fe₂O₃ + a-C/nc-Graphitschicht
Substrat+ TiO₂ + Al₂O₃ + TiO₂/Fe₂O₃+ a-C/nc-Graphitschicht
Substrat+ TiO₂ + MgO*SiO₂ + TiO₂/Fe₂O₃+ a-C/nc-Graphitschicht
Substrat+ TiO₂ + CaO*SiO₂ + TiO₂/Fe₂O₃+ a-C/nc-Graphitschicht
Substrat+ TiO₂ + Al₂O₃*SiO₂ + TiO₂/Fe₂O₃+ a-C/nc-Graphitschicht
Substrat+ TiO₂ + B₂O₃*SiO₂ + TiO₂/Fe₂O₃+ a-C/nc-Graphitschicht
Substrat+ TiO₂ + SiO₂+ a-C/nc-Graphitschicht
Substrat+ TiO₂ + SiO₂/Al₂O₃+ a-C/nc-Graphitschicht
Substrat+ TiO₂ + Al₂O₃+ a-C/nc-Graphitschicht
Substrat+ SnO₂ + a-C/nc-Graphitschicht
Substrat+ SnO₂ + TiO₂ + a-C/nc-Graphitschicht
Substrat+ SnO₂ + Fe₂O₃ + a-C/nc-Graphitschicht
Substrat+ SiO₂ + a-C/nc-Graphitschicht
Substrat+ SiO₂ + TiO₂ + a-C/nc-Graphitschicht
Substrat+ SiO₂ + TiO₂/Fe₂O₃ + a-C/nc-Graphitschicht
Substrat+ SiO₂ + Fe₂O₃ + a-C/nc-Graphitschicht
Substrat+ SiO₂ + TiO₂ + Fe₂O₃ + a-C/nc-Graphitschicht
Substrat+ SiO₂ + TiO₂ + Fe₃O₄ + a-C/nc-Graphitschicht
Substrat+ SiO₂ + TiO₂ + SiO₂ + TiO₂ + a-C/nc-Graphitschicht
Substrat+ SiO₂ + Fe₂O₃ + SiO₂ + TiO₂ + a-C/nc-Graphitschicht
Substrat+ SiO₂ + TiO₂/Fe₂O₃ + SiO₂ + TiO₂ + a-C/nc-Graphitschicht
Substrat+ SiO₂ + TiO₂ + SiO₂ + TiO₂/Fe₂O₃ + a-C/nc-Graphitschicht
Substrat+ SiO₂ + TiO₂ + SiO₂ + a-C/nc-Graphitschicht
Substrat+ SiO₂ + TiO₂ + SiO₂/Al₂O₃ + a-C/nc-Graphitschicht
Substrat+ SiO₂ + TiO₂ + Al₂O₃ + a-C/nc-Graphitschicht
Substrat + a-C/nc-Graphitschicht + TiO₂ + a-C/nc-Graphitschicht
Substrat + a-C/nc-Graphitschicht + Fe₂O₃ + a-C/nc-Graphitschicht
Substrat + a-C/nc-Graphitschicht + SiO₂ + SnO₂ + TiO₂ + a-C/nc-Graphitschicht
Substrat+ a-C/nc-Graphitschicht + SiO₂ + SnO₂ + TiO₂ + a-C/nc-Graphitschicht + TiO₂
Substrat + a-C/nc-Graphitschicht + SiO₂ + SnO₂ + TiO₂ + a-C/nc-Graphitschicht + Fe₂O₃

8. Pigmente nach einem oder mehreren der Ansprüche 1 bis 7, **dadurch gekennzeichnet, dass** die Pigmente aus 90 - 99 Gew.-% Partikel und 1 - 10 Gew.-% a-C/nc-Graphitschicht(en) bestehen, bezogen auf das Gesamtpigment.

9. Verfahren zur Herstellung der Pigmente nach einem oder mehreren der Ansprüche 1 bis 8, **dadurch gekennzeichnet, dass** Partikel in einem Wirbelschichtreaktor in einer Inertgasatmosphäre auf die gewünschte Reaktionstemperatur erhitzt werden und bei Erreichen der gewünschten Temperatur Kohlenstoff-Vorläufer zum Wirbelgas hinzugegeben werden und dass die Wirbelschichtreaktion nach der chemischen Abscheidung unter Inertgasatmosphäre bis zum Erreichen von Raumtemperatur abgekühlt wird.

10. Verfahren nach Anspruch 9, **dadurch gekennzeichnet, dass** die Kohlenstoff-Vorläufer aus der Gruppe von Zuckern und organischen Lösungsmitteln ausgewählt sind.

11. Verfahren nach den Ansprüchen 10 und 11, **dadurch gekennzeichnet, dass** es sich bei den Vorläufern um Ethanol, Isopropanol, Aceton, 2-Methyl-3-butin-2-ol, Puderzucker, Fructose, Glycose, Dextrose handelt.

12. Verfahren nach einem oder mehreren der Ansprüche 10 bis 12, **dadurch gekennzeichnet, dass** die Reaktionstemperatur bei 200 bis < 500 °C liegt.

13. Verfahren nach einem oder mehreren der Ansprüche 10 bis 13, **dadurch gekennzeichnet, dass** es sich bei dem Wirbelschichtreaktor um einen wirbelschichtgestützten CVD-Reaktor (FBCVD) handelt.

14. Verwendung der Pigmente nach einem oder mehreren der Ansprüche 1 bis 8 in Farben, Lacken, Autolacken, Autoreparaturlackierungen, Industrielacken, Pulverlacken, Druckfarben, Sicherheitsdruckfarben, Kunststoffen, keramischen Materialien, Kosmetika, Gläsern, Papier, Papierbeschichtung, Tonern für elektrophotographische Druckverfahren, Saatgut, Gewächshausfolien und Zeltplanen, wärmeleitenden, selbsttragenden, elektrisch isolierenden, flexiblen Folien zur Isolierung von Maschinen oder Vorrichtungen, als Absorber bei der Lasermarkierung von Papier und Kunststoffen, Absorber beim Laserschweißen von Kunststoffen, Pigmentanteigungen mit Wasser, organischen und/oder wässrigen Lösungsmitteln, in Pigmentzubereitungen und Trockenpräparaten.

15. Formulierung enthaltend Pigmente nach einem oder mehreren der Ansprüche 1 bis 8 in Mengen von 0,01 - 95 Gew.-%, bezogen auf die Gesamtformulierung.

16. Formulierung nach Anspruch 15, **dadurch gekennzeichnet, dass** sie zusätzlich mindestens eine Komponente enthält, der aus der Gruppe von Absorptionsmitteln, Adstringenzien, antimikrobiellen Stoffen, Antioxidantien, Antiperspiranzien, Antischaummitteln, Antischuppenwirkstoffen, Antistatika, Bindemitteln, biologischen Zusatzstoffen, Bleichmitteln, Chelatbildnern, Desodorierungsmitteln, Emollienzien, Emulgatoren, Emulsionsstabilisatoren, Farbstoffen, Feuchthaltemitteln, Filmbildnern, Füllstoffen, Geruchsstoffen, Geschmacksstoffen, Insektenrepellents, Konservierungsmitteln, Korrosionsschutzmitteln, kosmetischen Ölen, Lösungsmitteln, Wasser, Oxidationsmitteln, pflanzlichen Bestandteilen, Puffersubstanzen, Reduktionsmitteln, Tensiden, Treibgasen, Trübungsmitteln, UV-Filtern und UV-Absorbern, Vergällungsmitteln, Aloe vera, Avocadoöl, Coenzym Q10, Grüner-Tee-Extrakt, Viskositätsreglern, Parfüm und Vitaminen ausgewählt ist.

## Revendications

1. Pigments à base de particules, **caractérisées en ce que** les particules sont revêtues d'au moins une couche qui est constituée d'un mélange de carbone amorphe (a-C) et de graphite nanocristallin (nc-graphite), le terme « revêtues » signifiant que la surface respective des particules est entièrement revêtue/enveloppée.

2. Pigments selon la revendication 1, **caractérisés en ce que** le rapport a-C/nc-graphite se trouve dans la plage allant de 60 : 40 à 80 : 20.

3. Pigments selon la revendication 1 ou 2, **caractérisés en ce que** la couche d'a-C/nc-graphite présente une épaisseur de 1 - 10 nm.

4. Pigments selon l'une ou plusieurs parmi les revendications 1 à 3, **caractérisés en ce que** les particules sont choisies dans le groupe suivant de substrats : le mica naturel ou synthétique, le talc, le kaolin, les paillettes de Fe₂O₃, les paillettes de Fe₃O₄, les paillettes de Al₂O₃, les paillettes de BiOCI, les paillettes de verre, les paillettes de SiO₂, les paillettes de TiO₂, les paillettes de BN, les paillettes d'aluminium, les paillettes d'oxynitrure de Si, les paillettes de nitrure de Si/Ti et les paillettes de graphite, l'essence de perle, les paillettes synthétiques sans support, les billes de verre, les pigments de charge, les pigments d'interférence, les pigments multicouches, les pigments à double ton, les pigments goniochromatiques, les pigments à effet métallique, les particules de silicium ou des mélanges de ceux-ci.

5. Pigments selon l'une ou plusieurs parmi les revendications 1 à 4, **caractérisés en ce que** les substrats sont sphériques ou en forme de paillettes.

6. Pigments selon l'une ou plusieurs parmi les revendications 1 à 5, **caractérisés en ce que** les substrats sont revêtus par au moins un oxyde de métal et/ou un métal.

7. Pigments selon l'une ou plusieurs parmi les revendications 1 à 6, **caractérisés en ce que** les particules sont choisies dans le groupe suivant :
substrat+ couche d'a-C/nc-graphite
substrat+ couche d'a-C/nc-graphite + TiO₂
substrat+ couche d'a-C/nc-graphite + Fe₂O₃
substrat+ couche d'a-C/nc-graphite + Fe₃O₄
substrat+ couche d'a-C/nc-graphite + Cr₂O₃
substrat + couche d'a-C/nc-graphite + SiO₂
substrat + couche d'a-C/nc-graphite + ZrO₂
substrat+ couche d'a-C/nc-graphite + SnO₂
substrat+ couche d'a-C/nc-graphite + ZnO
substrat+ couche d'a-C/nc-graphite + Al
substrat+ couche d'a-C/nc-graphite + Fe
substrat+ couche d'a-C/nc-graphite + Cr
substrat+ TiO₂ + couche d'a-C/nc-graphite
substrat+ TiO₂/Fe₂O₃ + couche d'a-C/nc-graphite
substrat+ Fe₂O₃ + couche d'a-C/nc-graphite
substrat+ Fe₃O₄ + couche d'a-C/nc-graphite
substrat+ TiO₂ + Fe₂O₃ + couche d'a-C/nc-graphite
substrat+ TiO₂ + Fe₃O₄ + couche d'a-C/nc-graphite
substrat+ TiO₂ + SiO₂ + TiO₂ + couche d'a-C/nc-graphite
substrat+ TiO₂ + Al₂O₃ + TiO₂ + couche d'a-C/nc-graphite
substrat+ TiO₂ + MgO*SiO₂ + TiO₂ + couche d'a-C/nc-graphite
substrat+ TiO₂ + CaO*SiO₂ + TiO₂ + couche d'a-C/nc-graphite
substrat+ TiO₂ + Al₂O₃*SiO₂ + TiO₂ + couche d'a-C/nc-graphite
substrat+ TiO₂ + B₂O₃*SiO₂ + TiO₂ + couche d'a-C/nc-graphite
substrat+ Fe₂O₃ + SiO₂ + TiO₂ + couche d'a-C/nc-graphite
substrat+ Fe₂O₃ + Al₂O₃ + TiO₂ + couche d'a-C/nc-graphite
substrat+ Fe₂O₃ + MgO*SiO₂ + TiO₂ + couche d'a-C/nc-graphite
substrat+ Fe₂O₃ + CaO*SiO₂ + TiO₂ + couche d'a-C/nc-graphite
substrat+ Fe₂O₃ + Al₂O₃*SiO₂ + TiO₂ + couche d'a-C/nc-graphite
substrat+ Fe₂O₃ + B₂O₃*SiO₂ + TiO₂ + couche d'a-C/nc-graphite
substrat+ TiO₂/Fe₂O₃ + SiO₂ + TiO₂ + couche d'a-C/nc-graphite
substrat+ TiO₂/Fe₂O₃ + Al₂O₃ + TiO₂ + couche d'a-C/nc-graphite
substrat+ TiO₂/Fe₂O₃ + MgO*SiO₂ + TiO₂ + couche d'a-C/nc-graphite
substrat+ TiO₂/Fe₂O₃ + CaO*SiO₂ + TiO₂+ couche d'a-C/nc-graphite
substrat+ TiO₂/Fe₂O₃ + Al₂O₃*SiO₂ + TiO₂+ couche d'a-C/nc-graphite
substrat+ TiO₂/Fe₂O₃ + B₂O₃*SiO₂ + TiO₂+ couche d'a-C/nc-graphite
substrat+ TiO₂ + SiO₂ + TiO₂/Fe₂O₃ + couche d'a-C/nc-graphite
substrat + TiO₂/Fe₂O₃ + SiO₂ + TiO₂/Fe₂O₃ + couche d'a-C/nc-graphite
substrat + TiO₂/Fe₂O₃ + MgO*SiO₂ + TiO₂/Fe₂O₃ + couche d'a-C/nc-graphite
substrat+ TiO₂ + Al₂O₃ + TiO₂/Fe₂O₃+ couche d'a-C/nc-graphite
substrat+ TiO₂ + MgO*SiO₂ + TiO₂/Fe₂O₃+ couche d'a-C/nc-graphite
substrat+ TiO₂ + CaO*SiO₂ + TiO₂/Fe₂O₃+ couche d'a-C/nc-graphite
substrat+ TiO₂ + Al₂O₃*SiO₂ + TiO₂/Fe₂O₃+ couche d'a-C/nc-graphite
substrat+ TiO₂ + B₂O₃*SiO₂ + TiO₂/Fe₂O₃+ couche d'a-C/nc-graphite
substrat+ TiO₂ + SiO₂+ couche d'a-C/nc-graphite
substrat+ TiO₂ + SiO₂/Al₂O₃+ couche d'a-C/nc-graphite
substrat+ TiO₂ + Al₂O₃+ couche d'a-C/nc-graphite
substrat+ SnO₂ + couche d'a-C/nc-graphite
substrat+ SnO₂ + TiO₂ + couche d'a-C/nc-graphite
substrat+ SnO₂ + Fe₂O₃ + couche d'a-C/nc-graphite
substrat+ SiO₂ + couche d'a-C/nc-graphite
substrat+ SiO₂ + TiO₂ + couche d'a-C/nc-graphite
substrat+ SiO₂ + TiO₂/Fe₂O₃ + couche d'a-C/nc-graphite
substrat+ SiO₂ + Fe₂O₃ + couche d'a-C/nc-graphite
substrat+ SiO₂ + TiO₂ + Fe₂O₃ + couche d'a-C/nc-graphite
substrat+ SiO₂ + TiO₂ + Fe₃O₄ + couche d'a-C/nc-graphite
substrat+ SiO₂ + TiO₂+ SiO₂ + TiO₂ + couche d'a-C/nc-graphite
substrat+ SiO₂ + Fe₂O₃ + SiO₂ + TiO₂ + couche d'a-C/nc-graphite
substrat+ SiO₂ + TiO₂/Fe₂O₃ + SiO₂ + TiO₂ + couche d'a-C/nc-graphite
substrat+ SiO₂ + TiO₂+ SiO₂ + TiO₂/Fe₂O₃ + couche d'a-C/nc-graphite
substrat+ SiO₂ + TiO₂ + SiO₂ + couche d'a-C/nc-graphite
substrat+ SiO₂ + TiO₂ + SiO₂/Al₂O₃ + couche d'a-C/nc-graphite
substrat+ SiO₂ + TiO₂ + Al₂O₃ + couche d'a-C/nc-graphite
substrat + couche d'a-C/nc-graphite + TiO₂ + couche d'a-C/nc-graphite
substrat + couche d'a-C/nc-graphite + Fe₂O₃ + couche d'a-C/nc-graphite
substrat + couche d'a-C/nc-graphite + SiO₂ + SnO₂ + TiO₂ + couche d'a-C/nc-graphite
substrat + couche d'a-C/nc-graphite + SiO₂ + SnO₂ + TiO₂ + couche d'a-C/nc-graphite + TiO₂
substrat + couche d'a-C/nc-graphite + SiO₂ + SnO₂ + TiO₂ + couche d'a-C/nc-graphite + Fe₂O₃

8. Pigments selon l'une ou plusieurs parmi les revendications 1 à 7, **caractérisés en ce que** les pigments sont constitués de 90 - 99% en poids de particule et de 1 - 10% en poids de couche(s) d'a-C/nc-graphite, sur la base du pigment total.

9. Procédé de préparation des pigments selon l'une ou plusieurs parmi les revendications 1 à 8, **caractérisé en ce que** les particules sont chauffées dans un réacteur à lit fluidisé jusqu'à la température souhaitée dans une atmosphère de gaz inerte, et lorsque la température de réaction souhaitée est atteinte, des précurseurs de carbone sont ajoutés au gaz de fluidisation et **en ce que**, après le dépôt chimique, la réaction en lit fluidisé est refroidie dans une atmosphère de gaz inerte jusqu'à ce que la température ambiante soit atteinte.

10. Procédé selon la revendication 9, **caractérisé en ce que** les précurseurs de carbone sont choisis dans le groupe constitué par les sucres et les solvants organiques.

11. Procédé selon les revendications 10 et 11, **caractérisé en ce que** les précurseurs sont l'éthanol, l'isopropanol, l'acétone, le 2-méthyl-3-butin-2-ol, le sucre glace, le fructose, le glycose, le dextrose.

12. Procédé selon l'une ou plusieurs parmi les revendications 10 à 12, **caractérisé en ce que** la température de réaction va de 200 à < 500°C.

13. Procédé selon l'une ou plusieurs parmi les revendications 10 à 13, **caractérisé en ce que** le réacteur à lit fluidisé est un réacteur CVD en lit fluidisé (FBCVD).

14. Utilisation des pigments selon l'une ou plusieurs parmi les revendications 1 à 8, dans les peintures, les revêtements, les revêtements pour automobiles, les couches de finition pour automobiles, les revêtements industriels, les revêtements en poudre, les encres d'impression, les encres d'impression de sécurité, les matières plastiques, les matériaux céramiques, les cosmétiques, les verres, le papier, les revêtements de papier, les toners pour les procédés d'impression électro-photographique, les semences, les bâches et toiles pour serres, les feuilles flexibles thermiquement conductrices, autoporteuses, électriquement isolantes, pour l'isolement de machines ou de dispositifs, comme absorbant dans le marquage au laser de papier et de matières plastiques, comme absorbant dans le soudage au laser de matières plastiques, les pâtes de pigment avec de l'eau, des solvants organiques et/ou aqueux, dans les préparations de pigment et les préparations sèches.

15. Formulation contenant des pigments selon l'une ou plusieurs parmi les revendications 1 à 8, selon des quantités de 0,01 - 95% en poids, sur la base de la formulation dans son ensemble.

16. Formulation selon la revendication 15, **caractérisés en ce qu'**elle contient en outre au moins un composant choisi dans le groupe constitué par les absorbants, les astringents, les substances antimicrobiennes, les antioxydants, les antisudorifiques, les agents antimousse, les substances actives antipelliculaires, les antistatiques, les liants, les additifs biologiques, les agents de blanchiment, les agents chélatants, les agents désodorisants, les émollients, les émulsifiants, les stabilisateurs d'émulsion, les colorants, les humectants, les agents filmogènes, les charges, les fragrances, les arômes, les insectifuges, les conservateurs, les agents de protection contre la corrosion, les huiles cosmétiques, les solvants, l'eau, les oxydants, les constituants végétaux, les substances tampons, les agents réducteurs, les agents tensioactifs, les gaz propulseurs, les agents opacifiants, les filtres anti-UV et les absorbeurs UV, les agents dénaturants, l'aloe vera, l'huile d'avocat, le coenzyme Q10, l'extrait de thé vert, les régulateurs de viscosité, le parfum et les vitamines.
